# FASCICULE DE BREVET EUROPEEN

(11) **EP 3 937 948 B1**
(45) Date de publication et mention de la délivrance du brevet: **27.09.2023**
(21) Numéro de dépôt: 20709594.4
(22) Date de dépôt: 12.03.2020
(51) Int. Cl.: A61K 31/56, A61K 31/57, A61K 31/575, A61K 31/58, A61P 21/00

(54) **PHYTOECDYSONES ET LEURS DÉRIVÉS POUR LEUR UTILISATION DANS LE TRAITEMENT DE MALADIES NEUROMUSCULAIRES**
PHYTOECDYSONE UND IHRE DERIVATE ZUR VERWENDUNG BEI DER BEHANDLUNG NEUROMUSKULÄRER ERKRANKUNGEN
PHYTOECDYSONES AND THE DERIVATIVES THEREOF FOR USE IN THE TREATMENT OF NEUROMUSCULAR DISEASES

(30) Priorité: 15.03.2019 FR 1902726
(43) Date de publication de la demande: 19.01.2022
(73) Titulaire: Biophytis, 75001 Paris (FR); SORBONNE UNIVERSITE, 75006 Paris (FR)
(72) Inventeur: LATIL, Mathilde, 75019 Paris (FR); DILDA, Pierre, 75016 Paris (FR); LAFONT, René, 75016 Paris (FR); VEILLET, Stanislas, 91600 Savigny sur Orge (FR)
(74) Mandataire: Ipside
(86) Numéro de dépôt international: PCT/EP2020/056590
(87) Numéro de publication internationale: WO 2020/187678

(56) Documents cités:
- WO-A1-2015/177469
- WO-A1-2018/197708
- US-A1- 2016 220 624
- MOHSENI RASHIN ET AL: "Overexpression ofSMN2Gene in Motoneuron-Like Cells Differentiated from Adipose-Derived Mesenchymal Stem Cells by Ponasterone A", JOURNAL OF MOLECULAR NEUROSCIENCE, BIRKHAEUSER, CAMBRIDGE, MA, US, vol. 67, no. 2, 7 décembre 2018 (2018-12-07), pages 247-257, XP036699196, ISSN: 0895-8696, DOI: 10.1007/S12031-018-1232-X [extrait le 2018-12-07]
- LATIL M ET AL: "P.370BIO101 demonstrates combined beneficial effects on muscle and motor neurons in a mouse model of severe spinal muscular atrophy", NEUROMUSCULAR DISORDERS, ELSEVIER LTD, GB, vol. 29, 29 septembre 2019 (2019-09-29), XP085845071, ISSN: 0960-8966, DOI: 10.1016/J.NMD.2019.06.532 [extrait le 2019-09-29]

## Description

### Domaine technique de l'invention

L'invention relève de l'utilisation de phytoecdysones et de dérivés hémi-synthétiques de phytoecdysones pour le traitement de maladies neuromusculaires, en particulier l'amyotrophie spinale infantile.

### Technique antérieure

Les maladies neuromusculaires sont caractérisées par une altération du fonctionnement des unités motrices, composées de motoneurones, jonctions neuromusculaires et muscles squelettiques. Quelle que soit l'origine de la maladie, nerveuse comme dans l'amyotrophie spinale infantile ou la sclérose latérale amyotrophique, ou musculaire, toutes provoquent une altération de la fonction motrice des patients, pouvant aller du handicap à la mort prématurée lorsque les muscles vitaux sont atteints. La prise en charge de ces maladies neuromusculaires reste encore aujourd'hui symptomatique et représente un coût économique considérable au regard du niveau de handicap des patients, de la progressivité de ces pathologies et des besoins humains et matériels qu'elles nécessitent. La recherche et le développement de thérapies permettant d'alléger les symptômes moteurs et la dépendance des patients revêtent un intérêt social et économique certain.

Parmi ces maladies neuromusculaires, deux sont décrites comme affectant spécifiquement les motoneurones : les amyotrophies spinales infantiles (ou SMA), dont les symptômes apparaissent à l'enfance et la sclérose latérale amyotrophique (ou SLA) dont les symptômes se déclarent à l'âge adulte. Ces deux maladies neurodégénératives, aux causes et aux manifestations cliniques différentes, ont en commun une dénervation musculaire progressive, responsable d'une amyotrophie (AI-Chalabi et Hardiman, 2013 ; Crawford et Pardo, 1996).

Les amyotrophies spinales infantiles représentent la cause la plus commune de mortalité infantile d'origine génétique avec une prévalence de 1/6000 à 1/10000 naissances (Crawford et Pardo, 1996). Trois types principaux de sévérité sont décrits, suivant l'âge d'apparition des symptômes et la progression des atteintes cliniques, allant du type 1, le plus sévère, au type 3 dont la durée de vie peut être supérieure à 40 ans. Les patients atteints de SMA présentent une atteinte symétrique des muscles squelettiques par atrophie des fibres musculaires isolées ou regroupées en fascicules. La quasi-totalité des SMA sont à prédominance proximale, c'est-à-dire touchant les muscles du tronc et proches du tronc. Progressivement, le déficit moteur s'étend, dans un premier temps, aux muscles des membres inférieurs, puis dans un second temps, aux muscles des membres supérieurs en affectant préférentiellement les muscles extenseurs. Bien que présentant une grande hétérogénéité clinique, des analyses génétiques ont pu démontrer que toutes les formes de SMA sont causées par la mutation du gène *SMN1,* pour *Survival Of Motor Neurons,* positionné en région chromosomique 5q13. Dans le génome humain, il existe une copie centromérique inverse de ce gène, le gène *SMN2,* pouvant être retrouvé en plusieurs copies (Lorson et al., 1998), mais qui ne permet de compenser que partiellement la perte de fonction du gène *SMN1.* En effet, *SMN2* présente 5 différences nucléotidiques avec *SMN1,* dont une au niveau de l'exon 7, favorisant son excision par épissage dans 90% des ARNm produits par le gène *SMN2.* Cet épissage alternatif conduit à la production d'une protéine SMN^{Δ7} tronquée et instable. Ainsi, seuls 10% des protéines produites par le gène *SMN2* sont complètes et fonctionnelles (Vitte et al., 1997 ; Lefebvre et al., 1997). Il a donc été démontré un lien entre le nombre de copies du gène *SMN2,* leur niveau d'expression et la sévérité de la maladie. La protéine SMN est une petite protéine ubiquitaire, localisée dans des domaines discrets des noyaux, appelés Gems pour *Gemini of Coiled Bodies,* et dans le cytoplasme des cellules. Bien que la protéine SMN ait des rôles spécifiquement neuronaux, tels que la croissance axonale (McWhorter et al., 2003) et le transport axonal (Akten et al., 2011 ; Peter et al., 2011), elle est aussi impliquée dans des fonctions ubiquitaires telles que la biogénèse de ribonucléoprotéines nucléaires (Buhler et al., 1999 ; Liu et al., 1997 ; Zhang et al., 2008) ou encore la régulation de la traduction des ARNm (Sanchez et al., 2013). Ainsi, ni l'expression de la protéine SMN, ni ses fonctions cellulaires ne permettent d'expliquer directement la dégénérescence spécifique des motoneurones.

Cependant, le développement de différents modèles animaux et l'avancée de la recherche fondamentale ont permis de démontrer que la délétion conditionnelle du gène *Smn* uniquement au niveau des neurones ne reproduit pas les symptômes complets de la SMA, ni la dégénérescence motoneuronale (Frugier et al., 2000). D'autres études ont décrit un rôle de la protéine SMN dans le bon fonctionnement des astrocytes (Rindt et al., 2015), des cellules de Schwann (Hunter et al., 2014), du coeur (Bevan et al., 2010 ; Heier et al., 2010 ; Shabadi et al., 2010 ; Biondi et al., 2010), du foie (Vitte et al., 2004) et des vaisseaux (Somers et al., 2016), suggérant un rôle joué par le métabolisme énergétique dans la progression et la sévérité de la maladie. D'autres études encore se sont attachées à l'unité motrice dans la SMA et ont mis en évidence des altérations spécifiques des jonctions neuromusculaires (Kariya et al., 2008 ; Kong et al., 2009 ; Muray et al., 2008 ; Biondi et al., 2008), un constituant essentiel à la survie des motoneurones. En effet, il a été démontré que la protéine SMN participe au bon fonctionnement des jonctions neuromusculaires en agissant sur le pool de vésicules de neurotransmetteurs, l'activité synaptique (Torres-Benito et al., 2011), mais aussi la maturation des jonctions, étape cruciale permettant d'acquérir leur fonctionnalité et leur maintien (Kariya et al., 2008 ; Biondi et al., 2008). D'autre part, l'invalidation du gène *Smn* dans les cellules musculaires uniquement, engendre de graves altérations musculaires (Cifuentes-Diaz et al., 2001 ; Rajendra et al., 2007 ; Lee et al., 2011), telles qu'une dystrophie sévère (Cifuentes-Diaz et al., 2001), une mauvaise organisation de la structure sarcomérique permettant la contraction (Walker et al., 2008), une diminution de la fusion correcte des cellules souches musculaires, (Nicole et al., 2003), et donc de leur différentiation (Shafey et al., 2005). De plus, les cellules myogéniques des patients atteints de SMA sont pauvres en certains facteurs, comme les CANP (« *Calcium-Activated Neutral Protease* » en terminologie anglo-saxonne), essentiels à la mise en place des contacts neuromusculaires (Fidzianska et al., 1984 ; Vrbova et al., 1989). Enfin, il a été constaté, *in vitro,* que les motoneurones en co-culture avec des cellules musculaires SMA dégénèrent plus rapidement (Vrbova et al., 1989).

A ce jour, des approches très différentes ont été envisagées afin de développer un traitement pour la SMA. Les stratégies de recherches actuelles s'orientent, pour la plupart, sur la modification de l'expression de SMN directement ou non, soit par l'augmentation de l'expression du gène SMN2, ou la modification de l'épissage des transcrits SMN2, ou des thérapies géniques visant à réintroduire le gène correct. Certaines recherches se concentrent sur des approches indépendantes de SMN en développant des thérapies cellulaires ou bien en se focalisant sur l'activation de la neuroprotection (Olésoxime) ou sur l'amélioration de la fonction musculaire (activateur de troponine).

Malgré des dizaines d'essais cliniques en cours dans la SMA, à ce jour, seulement deux molécules ont été approuvées par les autorités.

La première, un activateur de la troponine (CK-2127107), vise à augmenter la sensibilité sarcomérique au calcium afin d'améliorer l'endurance et la performance de la fonction musculaire (Hwee et al., 2015). Cette molécule a récemment été désignée comme médicament orphelin pour la SMA par les agences règlementaires américaines (Mai 2017).

La seconde, un oligonucléotide anti-sens (ASO), vise à favoriser l'inclusion de l'exon 7 des transcrits produits par les gènes *SMN2,* permettant ainsi de faire surexprimer la protéine SMN complète par les cellules de la moelle épinière après injections intrathécales (Hache et al., 2016 ; Chiriboga et al., 2016). Cette molécule, développée par la société Biogen et nommée Nusinersen, a été approuvée par les autorités américaines et européennes et constitue la première molécule à visée thérapeutique mise sur le marché pour traiter les amyotrophies spinales infantiles sévères, de type 1 et de type 2. Les premiers résultats obtenus avec ASO ciblant l'intron 7, que ce soit sur modèles animaux (Hua et al., 2010 ; Passini et al., 2011) ou lors des essais cliniques de phases Il et III sur patients SMA sévères (Finkel et al., 2016), sont très impressionnants et prometteurs pour cette maladie létale. Cependant, plusieurs zones d'ombre subsistent quant à une utilisation d'un oligonucléotide *in vivo* injecté dans le système nerveux central seul et/ou sur le long terme. Tout d'abord, la communauté scientifique manque de recul sur la tolérance à long terme des patients pour ce genre de molécules exogènes ciblant l'expression génique. De plus, un autre questionnement concerne les risques inhérents à une surexpression importante et non contrôlée de la protéine SMN, protéine dont la fonction a été associée à la prolifération cellulaire (Grice et al., 2011). De plus, l'efficacité du Nusinersen semble d'autant plus importante que le début du traitement intervient précocement vis-à-vis de la progression de la maladie, situation clinique difficile à obtenir au regard du temps de diagnostic et du parcours clinique des familles. Enfin, dans la SMA, il est important de noter l'importance du rôle joué par les altérations multi-tissulaires sur la progression de la maladie. Or, le Nusinersen est une molécule qui ne franchit pas la barrière hémato-encéphalique, ce qui impose un traitement par voie intrathécale ne permettant de cibler que les neurones et les cellules gliales sans cibler les autres organes tels que les muscles, le foie, le pancréas et le système vasculaire. Leurs altérations pourraient, par conséquent, limiter les effets thérapeutiques du Nusinersen. Enfin, les patients atteints de SMA de type 3, pour qui le niveau d'expression de la protéine SMN est bien supérieur et pour lesquels le pronostic vital est rarement engagé, aucune autorisation de traitement n'a encore été approuvée, laissant cette sévérité orpheline de thérapie.

Il apparaît donc essentiel de développer des approches physiologiques et/ou pharmacologiques complémentaires au Nusinersen afin de potentialiser ses effets protecteurs et de limiter le plus possible l'évolution clinique des patients, leur dépendance et leur prise en charge clinique.

Les phytoecdysones représentent une importante famille de stérols polyhydroxylés. Ces molécules sont produites pas diverses espèces de plantes (fougères, gymnospermes, angiospermes) et participent à la défense de ces plantes contre les ravageurs. La phytoecdysone majoritaire du règne végétal est la 20-hydroxyecdysone.

Le brevet FR 3 021 318 divulgue que les phytoecdysones, et plus particulièrement la 20-hydroxyecdysone (20E), ont fait l'objet de nombreuses études pharmacologiques. Ces études ont mis en avant les propriétés antidiabétiques et anabolisantes de cette molécule. Ses effets stimulants sur les synthèses protéiques dans les muscles sont observés chez le rat *in vivo* (Syrov et al., 2000 ; Toth et al., 2008 ; Lawrence et al., 2012) et sur des myotubes murins C2C12 *in vitro* (Gorelick-Feldman et al., 2008). Certains des effets décrits ci-dessus dans des modèles animaux ont été retrouvés dans des études cliniques, encore peu nombreuses. Ainsi, la 20-hydroxyecdysone favorise l'augmentation de la masse musculaire chez de jeunes sportifs (Simakin et al., 1988). Enfin, le brevet français FR 3 021 318 décrit en outre l'utilisation de 20-hydroxyecdysone et de dérivés de 20-hydroxyecdysone, pour le traitement et la prévention de la sarcopénie et de l'obésité sarcopénique (Lafont et al., 2017).

MOHSENI RASHIN ET AL; JOURNAL OF MOLECULAR NEUROSCIENCE, vol. 67, no. 2, (2018-12-07), pages 247-257, divulgue l'utilisation de l'ecdysone Ponasterone A (PNA) pour favoriser la différenciation de cellules souches mésenchymateuses dérivées d'adipocytes en motoneurones dont le gène SMN2 est fonctionnel et permet d'exprimer la protéine SMN2, ce qui permet in fine de pallier le manque de protéine SMN2 dans le cadre de la SMA.

### Présentation de l'invention

La présente invention a pour objectif de limiter la perte motoneuronale liée à des maladies neuromusculaires ainsi que les conséquences de cette dégénérescence. Les phytoecdysones représentent une importante famille de phytostérols polyhydroxylés structuralement apparentés aux hormones de mue des insectes. Ces molécules sont produites par de nombreuses espèces végétales et participent à leur défense contre les insectes ravageurs. La phytoecdysone majoritaire est la 20-hydroxyecdysone.

Les inventeurs ont découvert de manière inattendue que la les phytoecdysones et des dérivés hémi-synthétiques de la phytoecdysones améliorent de manière significative la survie ainsi que l'évolution pondérale de mammifères atteints d'une amyotrophie spinale. De plus, les phytoecdysones et leurs dérivés hémi-synthétiques limitent l'atrophie et l'aplasie musculaire présentes dans cette pathologie et limitent significativement la perte des motoneurones des mammifères atteints par une amyotrophie spinale.

À cet effet, l'invention concerne une composition comprenant de la 20-hydroxyecdysone et/ou au moins un dérivé hémi-synthétique de 20-hydroxyecdysone, pour son utilisation dans le traitement d'une affection spécifique des motoneurones chez le mammifère atteint d'une maladie neuromusculaire comportant une altération de la fonction musculaire due à l'affection spécifique des motoneurones, la maladie neuromusculaire étant l'amyotrophie spinale infantile.

Dans des modes particuliers de réalisation, l'invention répond en outre aux caractéristiques suivantes, mises en oeuvre séparément ou en chacune de leurs combinaisons techniquement opérantes.

La 20-hydroxyecdysone et ses dérivés sont avantageusement purifiés au grade pharmaceutique.

La 20-hydroxyecdysone utilisée est de préférence sous forme d'un extrait de végétaux riches en 20-hydroxyecdysone ou d'une composition comportant à titre d'agent actif la 20-hydroxyecdysone. Des extraits de végétaux riches en 20-hydroxyecdysone sont par exemple des extraits de *Stemmacantha carthamoides* (aussi appelée *Leuzea carthamoides*)*, Cyanotis arachnoidea* et *Cyanotis vaga.*

Les extraits obtenus sont de préférence purifiés au grade pharmaceutique.

Dans un mode de réalisation la 20-hydroxyecdysone est sous forme d'extrait de plante ou d'une partie de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone. Ledit extrait est de préférence purifié au grade pharmaceutique. Ledit extrait est appelé par la suite BIO101. Il comporte de façon remarquable entre 0 et 0,05 %, en poids sec de l'extrait, d'impuretés, comme des composés mineurs, susceptibles d'affecter l'innocuité, la disponibilité ou l'efficacité d'une application pharmaceutique dudit extrait.

Selon un mode de réalisation de l'invention, les impuretés sont des composés à 19 ou 21 atomes de carbone, tels que la Rubrostérone, la Dihydrorubrostérone ou la Poststérone.

La plante à partir de laquelle est produit BIO101 est de préférence choisie parmi *Stemmacantha carthamoides* (aussi appelée *Leuzea carthamoides*)*, Cyanotis arachnoidea* et *Cyanotis vaga.*

Les dérivés de 20-hydroxyecdysone sont obtenus par hémisynthèse et peuvent notamment être obtenus de la façon décrite dans la demande de brevet européenne n° EP 15732785.9.

Selon un mode de réalisation préféré, l'altération de la fonction musculaire est due à une altération de la fonction des motoneurones ou leur dégénérescence.

Dans un mode de réalisation, la fonction musculaire altérée est celle du muscle strié squelettique.

Dans un mode de réalisation, l'altération de la fonction musculaire est liée à une aplasie et/ou une atrophie.

Dans un mode de réalisation particulier, l'affection des motoneurones résulte d'une altération génétique chez le mammifère atteint de la maladie neuromusculaire. Par altération génétique on entend une mutation telle qu'une substitution ou une insertion de nucléotide(s) ou une délétion de nucléotide(s).

L'invention vise la composition pour son utilisation chez le mammifère dans le traitement de l'amyotrophie spinale infantile (SMA).

Dans un mode de réalisation particulier, l'invention vise la composition pour son utilisation chez le mammifère dans le traitement d'une maladie neuromusculaire sporadique (liée à la mutation aléatoire d'un gène causal ou d'un ou plusieurs gènes de susceptibilité) ou d'une forme familiale dans laquelle on retrouve une mutation du gène SMN1 qui intervient dans le cadre de la SMA.

Dans un mode de réalisation particulier, le traitement de l'affection spécifique des motoneurones comporte l'amélioration de la survie motoneuronale et/ou l'accélération de la maturation des jonctions neuromusculaires.

Dans un mode de réalisation particulier, les phytoecdysones sont administrées à une dose comprise entre 3 et 15 milligrammes par kilogramme par jour chez l'humain. On entend ici par phytoecdysone, aussi bien les phytoecdysones de manière générale que leurs dérivés, la 20-hydroxyecdysone (notamment sous forme d'extrait) et ses dérivés.

De préférence, les phytoecdysones sont administrées à une dose de 200 à 1000 mg/jour, en une ou plusieurs prises, chez un humain adulte, et une dose de 5 à 350 mg/jour, en une ou plusieurs prises, chez l'humain enfant ou le nourrisson. On entend ici par phytoecdysone, aussi bien les phytoecdysones de manière générale que leurs dérivés, la 20-hydroxyecdysone (notamment sous forme d'extrait) et ses dérivés.

Dans des modes de réalisation la composition comporte au moins un composé considéré comme un dérivé de phytoecdysone, ledit au moins un composé étant de formule générale (I) : 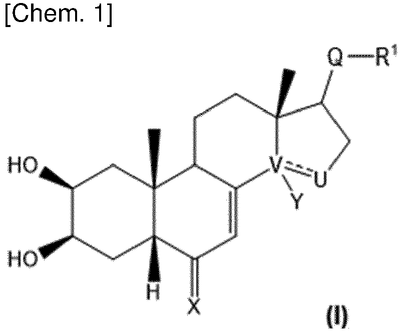 dans laquelle :
**V-U** est une liaison simple carbone-carbone et Y est un groupement hydroxyle :
**X** est un oxygène,
**Q** est un groupement carbonyle ;
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆) ; un groupement CH₂Br ;
**W** étant un hétéroatome choisi parmi N, O et S, de préférence O et encore plus préférentiellement S.

Dans le cadre de la présente invention on entend par « (C₁-C₆) », tout groupe alkyle de 1 à 6 atomes de carbones, linéaire ou ramifié, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle, éthyle, iso-propyle ou t-butyle, en particulier d'un groupe méthyle ou éthyle, plus particulièrement d'un groupe méthyle.

Dans un mode de réalisation préféré, dans la formule (I) :
**Y** est un groupement hydroxyle ;
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C6) ;
**W** étant un hétéroatome choisi parmi N, O et S, de préférence O et de préférence encore S.

Dans des modes de réalisation la composition comporte au moins un composé choisi parmi les composés suivants :
**n° 1** : (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one,
**n° 2** : (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
**n° 3** : (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
**n° 4** : (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
**n° 5** : (2S,3R,5R,10R,13R,14S,17S)-17-[2-(3-diméthylaminopropyl (méthyl)amino)acétyl]-2,3,14-trihydroxy-10,13-diméthyl-2,3,4,5,9,11,12,15,16,17-décahydro-1H-cyclopenta[a]phénanthrèn-6-one;
**n° 6** : 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
**n° 7** : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
**n° 8** : (2S,3R,5R,10R,13R,14S,17S)-2,3,14-trihydroxy-17-[2-(2-hydroxyéthyl sulfanyl)acétyl]-10,13-diméthyl-2,3,4,5,9,11,12,15,16,17-décahydro-1H cyclopenta[a]phénanthrèn-6-one.

Dans des modes de réalisation la composition comporte au moins un composé considéré comme un dérivé de phytoecdysone, ledit au moins un composé étant de formule générale (II) :

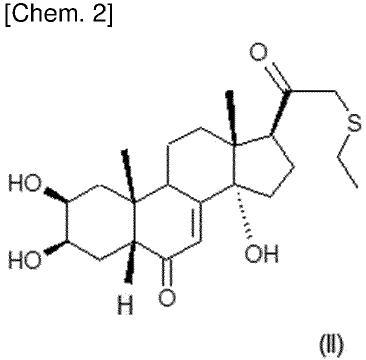

Le composé de formule (II) est par la suite appelé BIO103.

Dans des modes de réalisation la composition est incorporée à une formulation pharmaceutique acceptable pouvant être administrée par voie orale.

Dans le cadre de la présente invention on entend par « pharmaceutique acceptable » ce qui est utile dans la préparation d'une composition pharmaceutique, qui est généralement sûr, non toxique et qui est acceptable pour une utilisation vétérinaire de même que pharmaceutique humaine.

### Brève description des figures

L'invention sera mieux comprise à la lecture de la description suivante, donnée à titre d'exemple nullement limitatif, et faite en se référant aux figures qui représentent :
La figure 1A représente la courbe de l'évolution pondérale des souris SMA traitées avec un véhicule ou avec BIO101 de la naissance (P0) à 11 jours post-natal (P11). Ici et dans la suite de la description, P correspond au nombre de jours après la naissance (postnatal), n correspond à la taille de l'échantillon et p correspond à la « valeur p » utilisée pour quantifier la significativité statistique d'un résultat ;
La figure 1B est une représentation de Kaplan-Meier des courbes de survie pré-P11 des souris SMA traitées avec le véhicule ou avec BIO101 de P0 à P11 ;
La figure 2 est un histogramme représentant le nombre total de fibres musculaires d'un muscle *Tibialis anterior*(*Tibialis*) de souris contrôles saines (contrôle véhicule), SMA traitées avec le véhicule (SMA véhicule), ou SMA traitées avec BIO101 (SMA BIO101) de P0 à P11 ;
La figure 3A est une image représentative de section transversale du muscle *Tibialis anterior* colorée à l'Hématoxyline Eosine, de souris contrôle saine (contrôle) ;
La figure 3B est une image représentative de section transversale du muscle *Tibialis anterior* colorée à l'Hématoxyline Eosine, de souris SMA traitées avec le véhicule (SMA véhicule) de P0 à P11 ;
La figure 3C est une image représentative de section transversale du muscle *Tibialis anteriorcolorée* à l'Hématoxyline Eosine, de souris SMA traitées avec BIO101 (SMA BIO101) de P0 à P11 ;
La figure 3D est un histogramme représentant la surface de section transversale des fibres musculaires du muscle *Tibialis anterior (Tibialis)* de souris contrôles saines (contrôle véhicule), SMA traitées avec le véhicule (SMA véhicule) de P0 à P11, ou SMA traitées avec BIO101 (SMA BIO101) de P0 à P11 ;
La figure 3E est un histogramme représentant la surface de section transversale des fibres musculaires du muscle *Plantaris* de souris contrôles saines (contrôle véhicule), SMA traitées avec le véhicule (SMA véhicule) de P0 à P11, ou SMA traitées avec BIO101 (SMA BIO101) de P0 à P11 ;
La figure 3F est un histogramme représentant la surface de section transversale des fibres musculaires du muscle *Soleus* de souris contrôles saines (contrôle véhicule), SMA traitées avec le véhicule (SMA véhicule) de P0 à P11, ou SMA traitées avec BIO101 (SMA BIO101) de P0 à P11 ;
La figure 4A est un histogramme représentant la distribution des fibres musculaires du muscle *Tibialis anterior (Tibialis)* selon leur surface de section transversale, chez des souris SMA traitées avec le véhicule (SMA véhicule) de P0 à P11, ou SMA traitées avec BIO101 (SMA BIO101) de P0 à P11 ;
La figure 4B est un histogramme représentant la distribution des fibres musculaires du muscle *Plantaris* selon leur surface de section transversale, chez des souris SMA traitées avec le véhicule (SMA véhicule) de P0 à P11, ou SMA traitées avec BIO101 (SMA BIO101) de P0 à P11 ;
La figure 4C est un histogramme représentant la distribution des fibres musculaires du muscle *Soleus* selon leur surface de section transversale, chez des souris SMA traitées avec le véhicule (SMA véhicule) de P0 à P11, ou SMA traitées avec BIO101 (SMA BIO101) de P0 à P11 ;
La figure 5A représente un Western Blot illustrant la phosphorylation de AKT du muscle *Plantaris* de souris saines contrôles (CTL VH), de souris SMA traitées avec le véhicule (SMA VH) de P0 à P11, ou SMA traitées avec BIO101 (SMA BIO101) de P0 à P11 ;
La figure 5B représente un histogramme illustrant la quantification par densitométrie de la phosphorylation de AKT du muscle *Plantaris* de souris saines contrôles (CTL VH), de souris SMA traitées avec le véhicule (SMA VH) de P0 à P11, ou SMA traitées avec BIO101 (SMA BIO101) de P0 à P11 ;
La figure 5C représente un Western Blot illustrant la phosphorylation de ERK du muscle *Plantaris* de souris saines contrôles (CTL VH), de souris SMA traitées avec le véhicule (SMA VH) de P0 à P11, ou SMA traitées avec BIO101 (SMA BIO101) de P0 à P11 ;
La figure 5D représente un histogramme illustrant la quantification par densitométrie de la phosphorylation de ERK du muscle *Plantaris* de souris saines contrôles (CTL VH), de souris SMA traitées avec le véhicule (SMA VH) de P0 à P11, ou SMA traitées avec BIO101 (SMA BIO101) de P0 à P11 ;

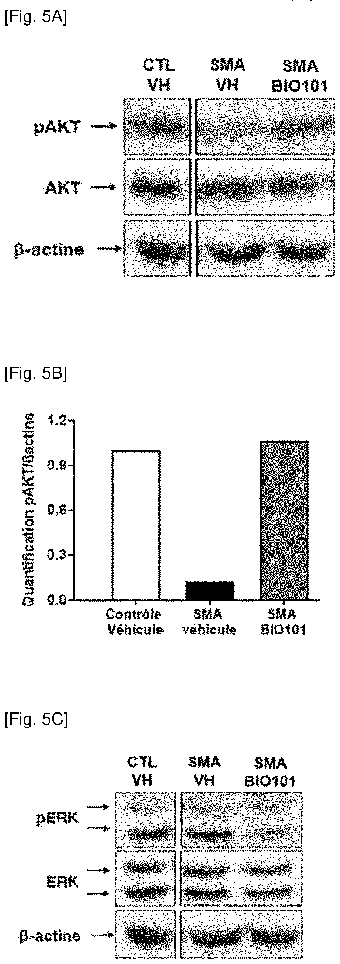

La figure 5C représente un Western Blot illustrant le niveau d'expression de la protéine SMN du muscle *Plantaris* de souris saines contrôles (CTL VH), de souris SMA traitées avec le véhicule (SMA VH) de P0 à P11, ou SMA traitées avec BIO101 (SMA BIO101) de P0 à P11 ;

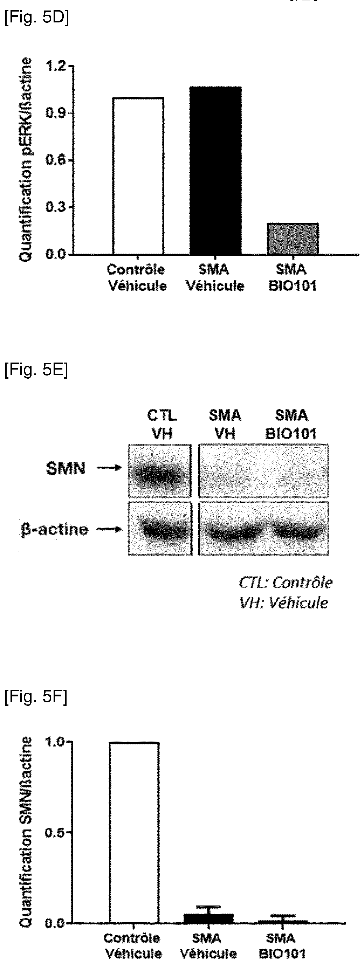

La figure 5F représente un histogramme illustrant la quantification par densitométrie de la protéine SMN du muscle *Plantaris* de souris saines contrôles (CTL VH), de souris SMA traitées avec le véhicule (SMA VH) de P0 à P11, ou SMA traitées avec BIO101 (SMA BIO101) de P0 à P11 ;

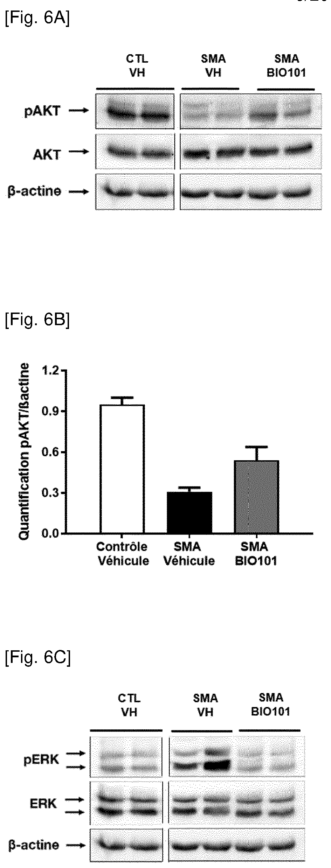

La figure 6A représente un Western Blot illustrant la phosphorylation de AKT au niveau de la moelle épinière de souris saines contrôles (CTL VH), de souris SMA traitées avec le véhicule (SMA VH) de P0 à P11, ou SMA traitées avec BIO101 (SMA BIO101) de P0 à P11 ;
La figure 6B représente un histogramme illustrant la quantification par densitométrie de la phosphorylation de AKT au niveau de la moelle épinière de souris saines contrôles (CTL VH), de souris SMA traitées avec le véhicule (SMA VH) de P0 à P11, ou SMA traitées avec BIO101 (SMA BIO101) de P0 à P11 ;
La figure 6C représente un Western Blot illustrant la phosphorylation de ERK au niveau de la moelle épinière de souris saines contrôles (CTL VH), de souris SMA traitées avec le véhicule (SMA VH) de P0 à P11, ou SMA traitées avec BIO101 (SMA BIO101) de P0 à P11 ;

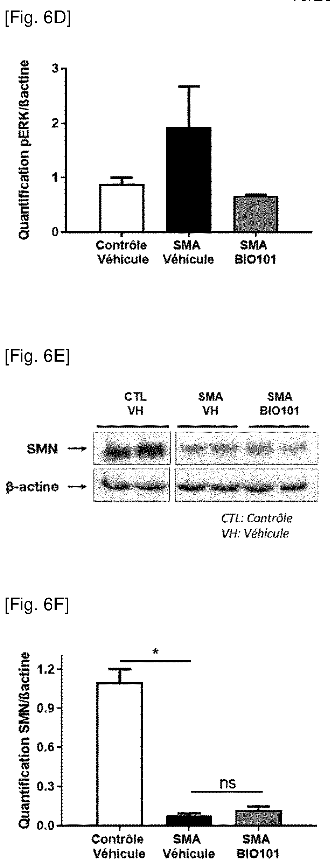

La figure 6D représente un histogramme illustrant la quantification par densitométrie de la phosphorylation de ERK au niveau de la moelle épinière de souris saines contrôles (CTL VH), de souris SMA traitées avec le véhicule (SMA VH) de P0 à P11, ou SMA traitées avec BIO101 (SMA BIO101) de P0 à P11 ;
La figure 6E représente un Western Blot illustrant le niveau d'expression de la protéine SMN au niveau de la moelle épinière de souris saines contrôles (CTL VH), de souris SMA traitées avec le véhicule (SMA VH) de P0 à P11, ou SMA traitées avec BIO101 (SMA BIO101) de P0 à P11 ;
La figure 6F représente un histogramme illustrant la quantification par densitométrie de la protéine SMN au niveau lombaire de la moelle épinière de souris saines contrôles (CTL VH), de souris SMA traitées avec le véhicule (SMA VH) de P0 à P11, ou SMA traitées avec BIO101 (SMA BIO101) de P0 à P11 ;

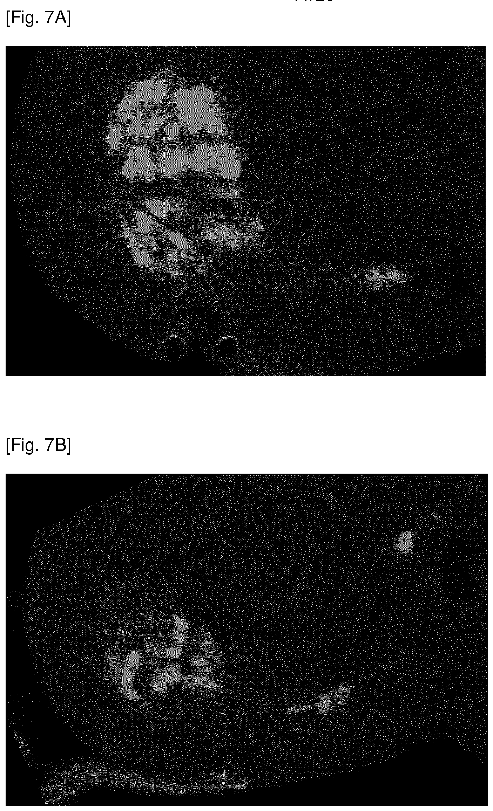

La figure 7A est une image représentative de marquage en immunofluorescence des motoneurones (anti-Choline Acétyltransférase) sur une coupe transversale de la région lombaire de la moelle épinière de souris contrôles saines (Contrôle Véhicule) ;
La figure 7B est une image représentative de marquage en immunofluorescence des motoneurones (anti-Choline Acétyltransférase) sur une coupe transversale de la région lombaire de la moelle épinière de souris SMA traitées au véhicule de P0 à P11 (SMA Véhicule) ;

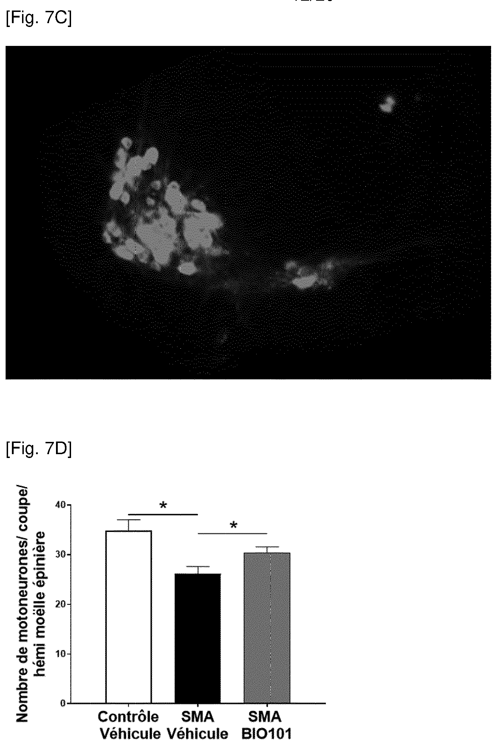

La figure 7C est une image représentative de marquage en immunofluorescence des motoneurones (anti-Choline Acétyltransférase) sur une coupe transversale de la région lombaire de la moelle épinière de souris SMA traitées au BIO101 de P0 à P11 (SMA BIO101) ;
La figure 7D représente un histogramme du nombre total de motoneurones par coupe par hémi moelle épinière ventrale de souris saines contrôles (contrôle véhicule), SMA traitées au véhicule (SMA véhicule) de P0 à P11, SMA traitées avec BIO101 (SMA BIO101) de P0 à P11 ;

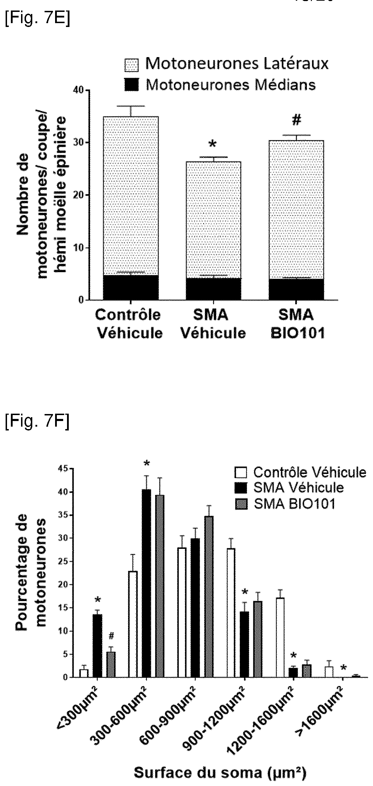

La figure 7E représente un histogramme du nombre de motoneurones latéraux et distaux par coupe par hémi moelle épinière ventrale de souris saines contrôles (contrôle véhicule), SMA traitées au véhicule (SMA véhicule) de P0 à P11, SMA traitées avec BIO101 (SMA BIO101) de P0 à P11 ;
La figure 7F est un histogramme représentant la distribution des motoneurones selon leur surface de soma, par coupe par hémi moelle épinière ventrale de souris saines contrôles (contrôle véhicule), SMA traitées au véhicule (SMA véhicule) de P0 à P11, SMA traitées avec BIO101 (SMA BIO101) de P0 à P11 ;

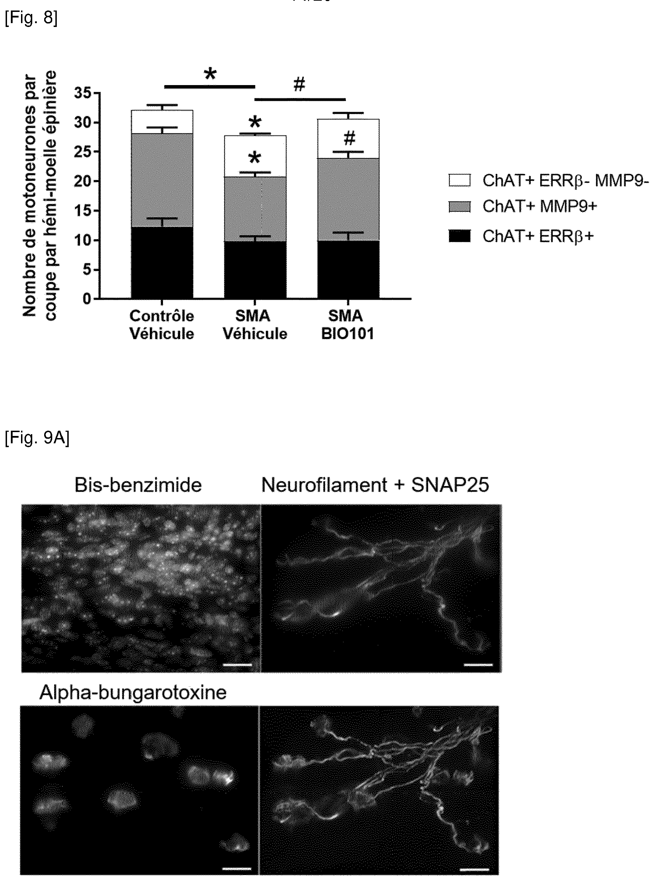

La Figure 8 est un histogramme représentant la répartition du nombre des différents types de motoneurones (lents, intermédiaires et rapides) dans les groupes de souris contrôles saines (contrôle véhicule n≥5), SMA traitées avec le véhicule (SMA véhicule, n=5) de P0 à P11, ou SMA traitées avec BIO101 (SMA BIO101, n≥6) de P0 à P11 ; Les motoneurones lents sont caractérisés par l'expression des marqueurs ChAT+ERRβ+, les motoneurones rapides par l'expression des marqueurs ChAT+MMP9+ et les motoneurones intermédiaires par l'expression des marqueurs ChAT+ERRβ-MMP9-. La significativité est considérée atteinte pour p<0.05. "*" indique une différence significative comparée au groupe de souris contrôles véhicule. "#" indique une différence significative comparée au groupe de souris SMA véhicule.
La Figure 9A représente des photos de marquage en immunofluorescence de la jonction neuromusculaire par les différents marqueurs de la face pré-synaptique (Neurofilament et SNAP25) et de la face post-synaptique (α-bungarotoxine). Le marquage au bis-benzimide permet d'identifier les noyaux. La barre d'échelle représente 10µm.

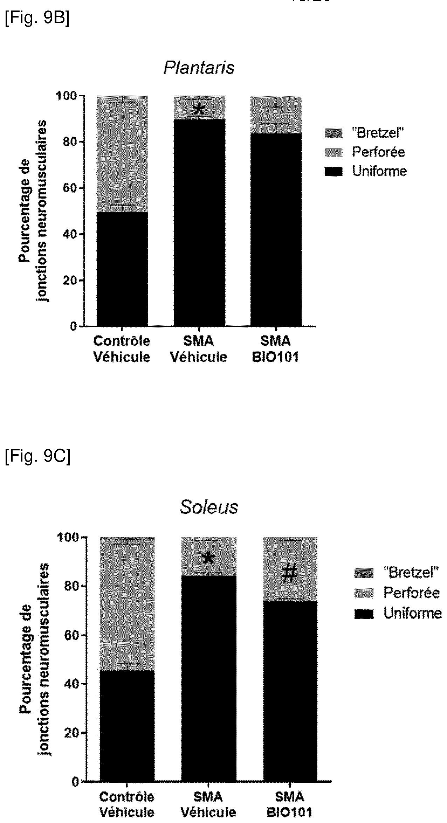

La Figure 9B est un histogramme représentant la répartition du pourcentage des différents types de jonctions neuromusculaires en fonction de leur état de maturation (de forme dite « bretzel » : matures ; perforées : en cours de maturation ;
ou uniformes : immatures) dans le muscle *Plantaris* dans les groupes de souris contrôles saines (contrôle véhicule, n=4), SMA traitées avec le véhicule (SMA véhicule, n=4) de P0 à P11, ou SMA traitées avec BIO101 (SMA BIO101, n=4) de P0 à P11. "*" indique une différence significative (p<0,05) comparée au groupe de souris contrôles véhicule.
La Figure 9C est un histogramme représentant la répartition du pourcentage des différents types de jonctions neuromusculaires en fonction de leur état de maturation (de forme dite « bretzel » : matures ; perforées : en cours de maturation ;
ou uniformes : immatures) dans le muscle *Soleus* dans les groupes de souris contrôles saines (contrôle véhicule, n=4), SMA traitées avec le véhicule (SMA véhicule, n=4) de P0 à P11, ou SMA traitées avec BIO101 (SMA BIO101, n=4) de P0 à P11. "*" indique une différence significative (p<0,05) comparée au groupe de souris contrôles véhicule. "#" indique une différence significative comparée au groupe de souris SMA véhicule.

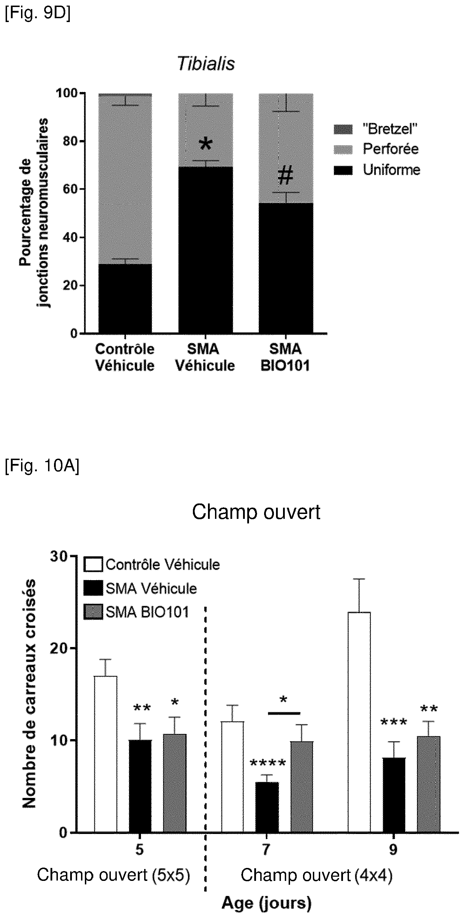

La Figure 9D est un histogramme représentant la répartition du pourcentage des différents types de jonctions neuromusculaires en fonction de leur état de maturation (de forme dite « bretzel » : matures ; perforées : en cours de maturation ;
ou uniformes : immatures) dans le muscle *Tibialis anterior* dans les groupes de souris contrôles saines (contrôle véhicule, n=4), SMA traitées avec le véhicule (SMA véhicule, n=4) de P0 à P11, ou SMA traitées avec BIO101 (SMA BIO101, n=4) de P0 à P11. "*" indique une différence significative (p<0,05) comparée au groupe de souris contrôles véhicule. "#" indique une différence significative comparée au groupe de souris SMA véhicule.
La Figure 10A représente les performances motrices évaluées par le test *Open field* aux jours P5, P7 et P9 des souris contrôles saines (contrôle véhicule, n=13), SMA traitées avec le véhicule (SMA véhicule, n=11) de P0 à P9, ou SMA traitées avec BIO101 (SMA BIO101, n=12) de P0 à P9. Le nombre de "*" indique la différence significative comparée au groupe de souris contrôles véhicule (*=p<0,05, **=p<0,01, ***=p<0,001 et ****=p<0,0001).

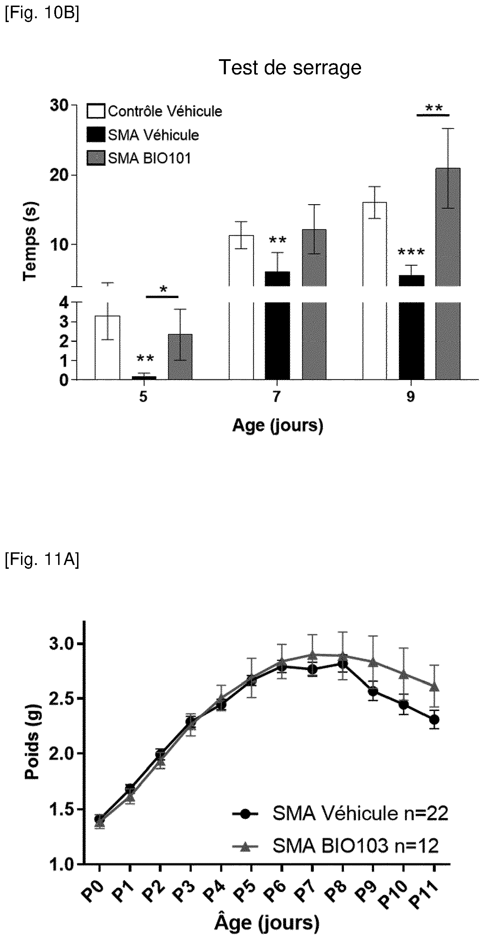

Figure 10B représente la fatigabilité musculaire évaluée par le grip test aux jours P5, P7 et P9 des souris contrôles saines (contrôle véhicule, n=17), SMA traitées avec le véhicule (SMA véhicule, n=11) de P0 à P9, ou SMA traitées avec BIO101 (SMA BIO101, n=10) de P0 à P9. Le nombre de "*" indique la différence significative comparée au groupe de souris contrôles véhicule (*=p<0,05, **=p<0,01 et ***=p<0,001).
La figure 11A représente la courbe de poids des souris SMA traitées avec le véhicule ou avec BIO103 de P0 à P11 ;

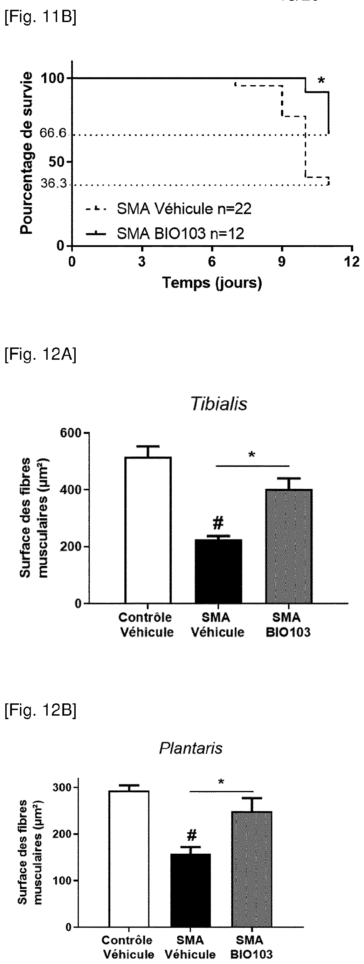

La figure 11B est une représentation de Kaplan-Meier des courbes de survie pré-P11 des souris SMA traitées avec le véhicule ou avec BIO101 de P0 à P11 ;
La figure 12A est un histogramme représentant la surface de la section transversale des fibres musculaires du muscle *Tibialis anterior (Tibialis)* de souris contrôles saines (contrôle véhicule), SMA traitées avec le véhicule (SMA véhicule) de P0 à P11, ou SMA traitées avec BIO103 (SMA BIO101) de P0 à P11 ;
La figure 12B est un histogramme représentant la surface de section transversale des fibres musculaires du muscle *Plantaris* de souris contrôles saines (contrôle véhicule), SMA traitées avec le véhicule (SMA véhicule) de P0 à P11, ou SMA traitées avec BIO103 (SMA BIO101) de P0 à P11 ;

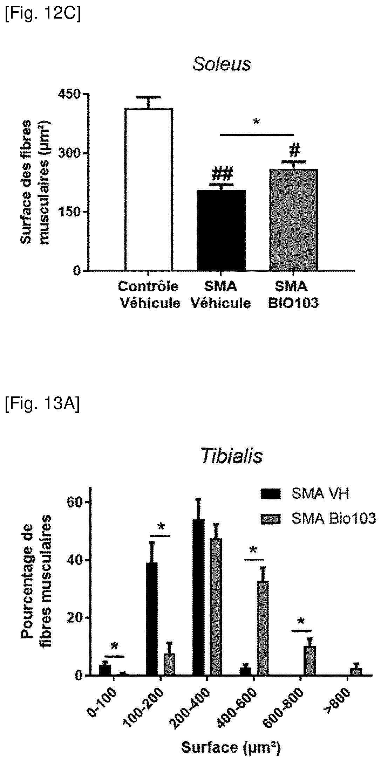

La figure 12C est un histogramme représentant la surface de section transversale des fibres musculaires du muscle *Soleus* de souris contrôles saines (contrôle véhicule), SMA traitées avec le véhicule (SMA véhicule) de P0 à P11, ou SMA traitées avec BIO103 (SMA BIO101) de P0 à P11 ;
La figure 13A est un histogramme représentant la distribution des fibres musculaires du muscle *Tibialis anterior (Tibialis)* selon la surface de section transversale, de souris SMA traitées avec le véhicule (SMA véhicule) de P0 à P11, ou SMA traitées avec BIO103 (SMA BIO103) de P0 à P11 ;

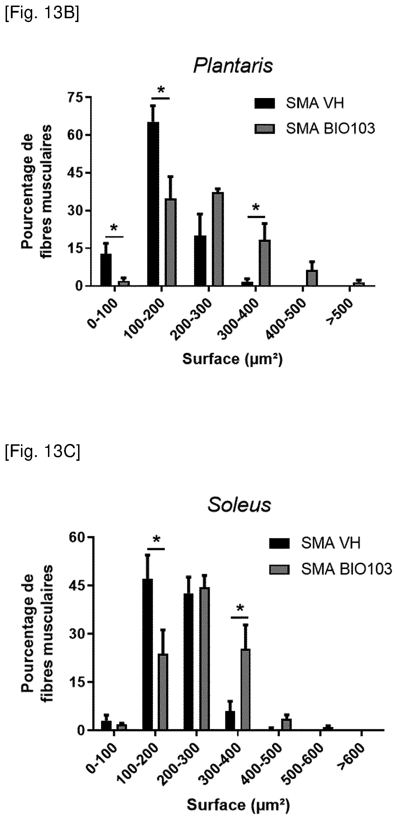

La figure 13B est un histogramme représentant la distribution des fibres musculaires du muscle *Plantaris* selon la surface de section transversale, de souris SMA traitées avec le véhicule (SMA véhicule) de P0 à P11, ou SMA traitées avec BIO103 (SMA BIO103) de P0 à P11 ;
La figure 13C est un histogramme représentant la distribution des fibres musculaires du muscle *Soleus* selon la surface de section transversale, de souris SMA traitées avec le véhicule (SMA véhicule) de P0 à P11, ou SMA traitées avec BIO103 (SMA BIO103) de P0 à P11 ;

### Description des modes de réalisation

L'invention sera décrite ci-après dans le contexte particulier de certains de ses domaines d'application préférés, non limitatifs.

### 1. Procédé de purification de BIO101

BIO101 est préparé à partir de 20-hydroxyecdysone pure à de l'ordre de 90%, selon les étapes suivantes :
i) dissolution à chaud de 20-hydroxyecdysone pure à de l'ordre de 90% dans du méthanol, filtration et concentration partielle,
ii) ajout de 3 volumes d'acétone,
iii) refroidissement à une température comprise entre 0 et 5°C, sous agitation,
iv) filtration du précipité obtenu,
v) rinçages successifs avec de l'acétone et de l'eau, et
vi) séchage.

Cette purification met en jeu un processus de recristallisation approprié à cette molécule et apte à être réalisé à l'échelle industrielle.

La filtration de l'étape i) est réalisée grâce à un filtre à particules de 0,2 µm.

La concentration partielle de l'étape i) est avantageusement effectuée par distillation sous vide, à une température de l'ordre de 50°C, en présence de MeOH.

L'étape vi) de séchage est effectuée sous vide à une température de l'ordre de 50°C.

### 2. Procédé de synthèse de BIO103

BIO103 est obtenu par hemi-synthèse à partir de 20-hydroxyecdysone puis purification au grade pharmaceutique selon le procédé de préparation suivant :

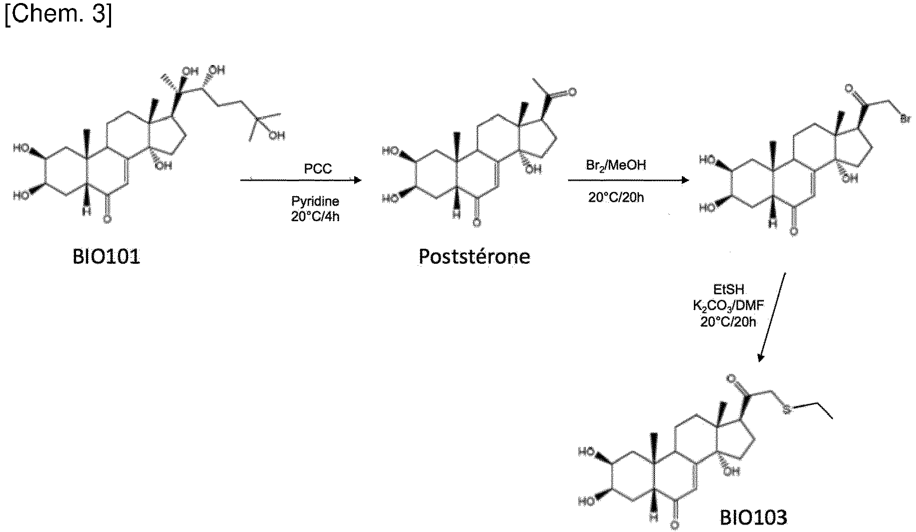

Schéma de synthèse de BIO103 en 3 étapes :
I) coupure oxydante de la chaîne latérale de la 20-hydroxyecdysone entre les carbones C20 et C22 pour obtenir la poststérone (protocole connu de l'homme du métier),
II) introduction d'un atome de brome en position C21,
III) réaction du composé bromé ainsi obtenu avec de l'éthane-thiol.

### 3. Activité biologique de BIO101 et BIO103

### a. Analyse phénotypique des effets de BIO101

Un modèle de souris SMA sévère a été utilisé sur fonds génétique FVB/NRj, caractérisé par l'invalidation de l'exon 7 du gène murin *Smn* et exprimant 2 copies du transgène humain *SMN2 (Smn*^{*Δ7*/}*^{Δ7}; huSMN2*^{*+*/*+*}) (Hsieh et al., 2000). Les souris issues de ces croisements ayant le génotype « FVB/NRj-Smn^{Δ7/Δ7} huSMN2^{+/+} 2 copies » sont décrites comme « SMA ». Ces souris se caractérisent par un défaut de croissance progressif qui s'observe à partir de 4 jours post-natal et présentent une dégénérescence d'environ 50% des motoneurones de la corne ventrale de la moelle épinière en fin de vie, une atrophie musculaire progressive et une durée de vie moyenne de 12 jours environ (Hsieh et al., 2000). Les souris ayant le génotype « FVB/NRj-Smn^{+/Δ7} huSMN2^{+/+} 2 copies » ne présentent aucun phénotype particulier et sont utilisées comme souris dites « contrôles ». Les souris ont été traitées par gavage quotidien à une dose de 50mg/kg, soit avec la molécule BIO101 complexée à un véhicule (dans le cas présent la cyclodextrine), soit avec le véhicule seul (VH). Le poids et la survie ont été analysés quotidiennement jusqu'à P11. Dans la suite de la description, n correspond à la taille de l'échantillon et p correspond à la « valeur p » utilisée pour quantifier la significativité statistique d'un résultat.

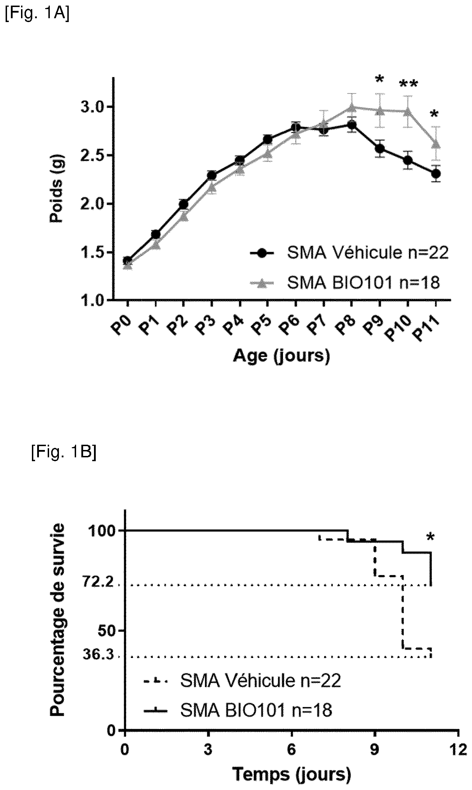

Les résultats démontrent que BIO101 seul (n=18, avec n pour la taille de l'échantillon), en traitement oral quotidien dès la naissance des souris, permet de limiter significativement (p<0,05) la perte de poids des animaux visible dès 9 jours post-natal en comparaison à des animaux traités avec le véhicule (n=22) (**Figure 1A****)** et diminue de manière significative (p<0,05) la mortalité pré-P11 des animaux traités (**Figure 1B**).

### b. Analyse des effets trophiques musculaires de BIO101

À P11, une heure après leur dernier gavage, les souris traitées ont été anesthésiées avec du pentobarbital 1% à 6µL/g de souris puis les muscles extenseur *Soleus,* à typologie mixte, extenseur *Plantaris,* à typologie rapide, et fléchisseur *Tibialis,* à typologie rapide, ont été prélevés pour réaliser des études histologiques ou moléculaires.

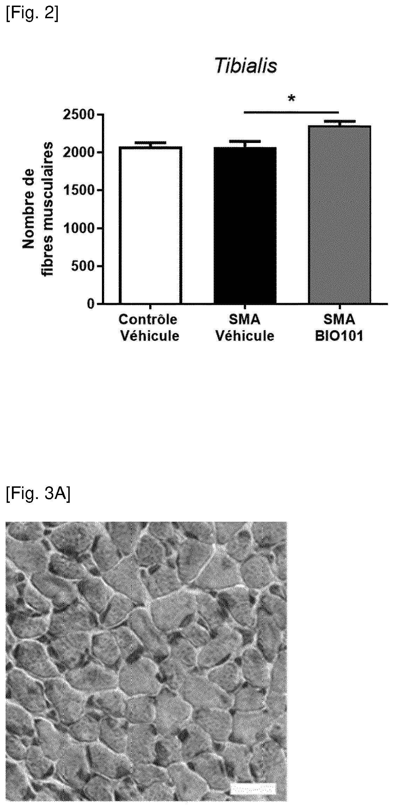

Après prélèvement, les muscles *Soleus, Plantaris* et *Tibialis* ont été inclus individuellement dans un milieu de conservation puis congelés dans l'isopentane refroidi. Pour chaque muscle, des sections médiales transverses de 10µm d'épaisseur ont été réalisées. Ces sections ont été colorées à l'Hématoxyline-Eosine, déshydratées et montées dans un milieu d'inclusion. Les images de ces sections ont été réalisées avec un microscope (avec un grossissement x200). Afin d'obtenir des images avec une impression de relief, la technique de contraste interférentiel différentiel a été utilisée. A partir de ces images, le nombre de fibres musculaires de chaque muscle et la surface de section transverse de 20% de ces fibres ont été comptées en utilisant un logiciel de traitement d'image (**Figure 2**). L'analyse histologique des coupes transversales des muscles prélevés colorées à l'Hématoxyline-Eosine, montre un effet bénéfique de BIO101 sur l'aplasie musculaire (nombre de fibres musculaires). En effet, dans le *Tibialis,* le nombre de fibres musculaires des souris SMA traitées avec BIO101 augmente significativement comparé au nombre de fibres des animaux traités avec le véhicule (respectivement 2358 et 2069 fibres (+14%), p<0,05).

De manière intéressante, l'atrophie musculaire présente chez les souris SMA est limitée par le traitement, quelles que soient la nature et la typologie des muscles. En effet, le traitement par BIO101 permet de limiter significativement l'atrophie des trois muscles étudiés : fléchisseur ou extenseur.

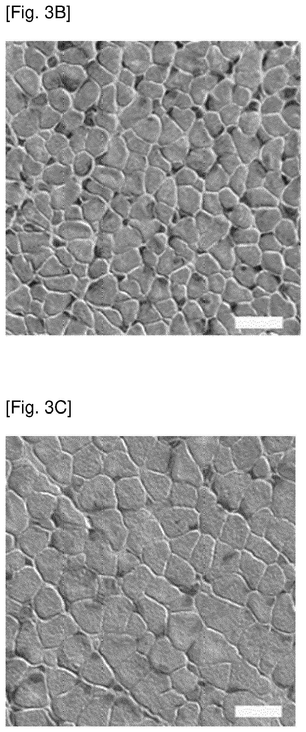

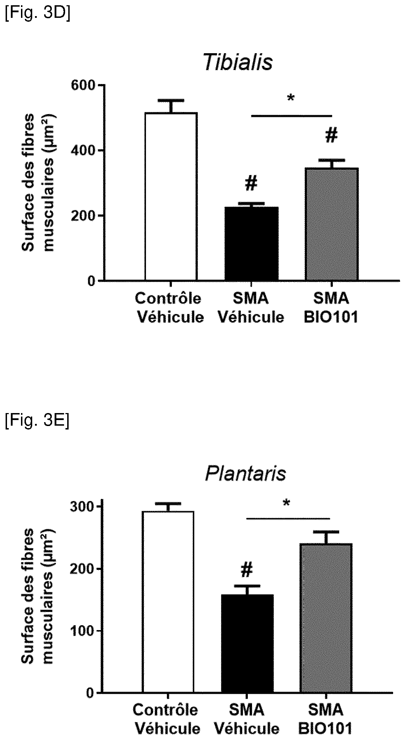

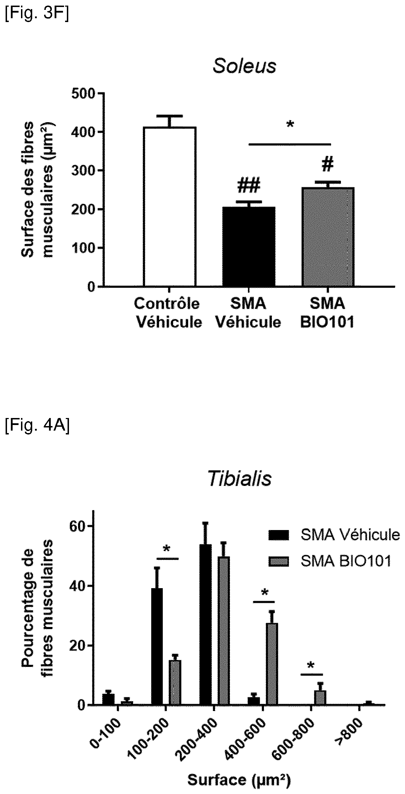

L'atrophie des fibres musculaires dans un contexte SMA (**Figure 3A**) par rapport à des souris saines contrôles (**Figure 3B**) est bien visible sur les coupes histologiques de *Tibialis* et la quantification de la surface de section transversale des fibres musculaires montre que cette atrophie est de 56,1% dans le *Tibialis* de souris SMA (n=4) par rapport à des souris contrôles (n=4) (**Figure 3D**). Cette atrophie est réduite significativement (p<0,05) à 32,7% à P11 grâce à un traitement avec BIO101 (n=4) (**Figure 3C** et **Figure 3D**)**.** Il en est de même pour le *Plantaris* où l'on observe une atrophie significative des fibres musculaires de 46% chez les souris SMA par rapport aux souris saines contrôles (p<0,05) et où le traitement par BIO101 limite significativement cette atrophie à 17,9% (p<0,05) (**Figure 3E**) et dans le *Soleus* (50,2% pour des souris SMA comparées au contrôle (p<0,01) versus 37,8% avec un traitement avec BIO101, p<0,05) (**Figure 3F**).

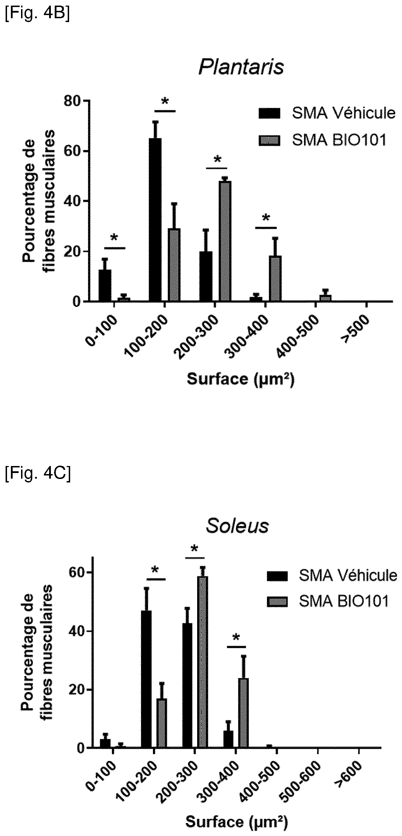

Afin de réaliser une analyse plus fine des effets de BIO101 sur l'atrophie, la distribution des fibres musculaires par catégories de surface de section transversale a été évaluée. Selon leur nature, les fibres musculaires ont une surface de section transversale différente. Les fibres de type I, caractérisées par une contraction lente, ont une taille inférieure à celle des fibres de type II, caractérisées par une contraction rapide. La réduction significative de l'atrophie par la molécule BIO101 observée avec un traitement des souris SMA de P0 à P11, se traduit par un effet significatif de ce traitement sur la distribution des fibres selon leur surface de section transversale dans les trois muscles étudiés des souris SMA traitées avec BIO101 en comparaison avec les souris SMA traitées avec le véhicule (**Figures 4A****,** **4B et 4C**)**.** Dans le muscle *Tibialis* des souris SMA traitées avec BIO101 de P0 à P11, on observe une augmentation significative de la proportion de fibres ayant une surface de section transversale comprise entre 400 et 800 µm² tandis que la proportion de fibres ayant une surface de section transversale comprise entre 100 et 200 µm² diminue significativement (p<0,05) par rapport au groupe de souris SMA véhicule (**Figure 4A**)**.** Dans le muscle *Plantaris* et dans le *Soleus* des SMA traitées avec BIO101 de P0 à P11, on observe une augmentation de la proportion de fibres ayant une surface de section transversale entre 200 et 400 µm² accompagnée d'une diminution de celles ayant une surface de section transversale entre 0 et 100 µm² pour le muscle *Plantaris* (**Figure 4B**) et de celles ayant une surface de section transversale entre 100 et 200 µm² pour le *Soleus* (**Figure 4C**)**.**

### c. Analyses moléculaires

Des études antérieures ont permis de démontrer une sous-activation de la voie AKT/CREB et une sur-activation de la voie ERK/Elk-1 associées à une faible expression de la protéine SMN au niveau de la corne ventrale de la moelle épinière des souris transgéniques « FVB/NRj-Smn^{Δ7/Δ7} huSMN2^{+/+} 2 copies » utilisées comme modèle murin sévère d'amyotrophie spinale de type II, suggérant un rôle de ces voies dans la maladie (Branchu et al., 2013).

Le muscle *Plantaris* congelé a été homogénéisé dans du tampon d'extraction par un broyage mécanique. Le surnageant contenant l'extrait protéique a été prélevé, puis les extraits protéiques ont ensuite été dosés selon la méthode de Lowry. Une électrophorèse sur gel SDS-PAGE a été réalisée, puis les protéines séparées ont été transférées sur membranes. Les anticorps primaires utilisés sont les suivants : monoclonal de souris anti-SMN (1 :5000), polyclonal de lapin anti-Ser 473 phospho-AKT (1:1000), polyclonal de lapin anti-AKT (1/100), monoclonal de lapin anti phospho-ERK 1/2 (1 :500), anti-MAP Kinase 1/2 (ERK 1/2) (1 :1000). Après avoir été rincées, les membranes ont été incubées avec des anticorps secondaires anti souris (1:5000) ou anti lapin (1:5000) conjugués à la peroxydase. Après l'utilisation des anticorps primaires, les complexes anticorps-antigènes ont été rompus par incubation dans une solution de dissociation puis les membranes sont incubées de nouveau avec les anticorps de lapin anti-AKT et anti-MAP Kinase 1/2 (ERK 1/2) (1 :1000). Les complexes d'anticorps ont été révélés par chimioluminescence et ont été imagés par un appareil d'acquisition d'images numériques d'un échantillon de gel, de membrane ou de film. La densité optique de chaque bande spécifique a quant à elle été quantifiée avec un logiciel de traitement d'image en soustrayant le fond et en normalisant avec la densité optique des bandes de β-actine. Pour les contrôles traités avec le véhicule, les valeurs obtenues ont été déterminées à 1 et les valeurs des autres groupes ont été normalisées par rapport à ces contrôles et exprimées en quantité relative. Les animaux issus de chaque groupe ont été obtenus en réalisant des expériences indépendantes et en utilisant différentes membranes sur lesquelles chaque groupe a été comparé avec les contrôles. Les valeurs quantitatives de pAKT au niveau du *Plantaris* représentent 3 souris par groupe traité avec le véhicule (contrôle ou SMA) et 4 souris traitées avec BIO101. Les valeurs quantitatives de pERK au niveau du *Plantaris* représentent au moins 4 souris par groupe. Les valeurs quantitatives de SMN représentent n=2. Concernant les valeurs quantitatives de pAKT au niveau de la moelle épinière, elles représentent n=2 souris par groupe pour pAKT et pERK, tandis qu'elles représentent n=4 par groupe pour le niveau de SMN.

L'analyse moléculaire dans le muscle *Plantaris* confirme que la voie AKT est bien sous-activée (**Figures 5A et 5B**) au profit de la voie ERK (**Figures 5C** **et** **5D**) chez les souris SMA par rapport aux souris contrôles véhicules (CTL VH) (Branchu et al., 2013). Le traitement de 11 jours avec BIO101 permet d'inverser ce rapport dans les muscles des souris traitées de P0 à P11 en augmentant très fortement la phosphorylation de AKT (pAKT) (**Figure 5B**) tandis que le niveau de phosphorylation de ERK (pERK) est très diminué (**Figure 5D**). Cette situation bénéfique et déjà démontrée comme impliquée dans la surexpression de la protéine SMN (Branchu et al., 2013). Comme attendu, la protéine SMN n'est quasiment pas exprimée chez des animaux SMA par rapport à des animaux contrôles sains. Néanmoins, et de manière tout à fait étonnante, aucune modification du niveau d'expression de SMN n'a pu être observée après un traitement avec BIO101, suggérant une régulation moléculaire originale (**Figures 5E et 5F**).

Cette balance des voies de signalisation entre ERK et AKT est également retrouvée au niveau de la moelle épinière des souris SMA. En effet, on observe une franche diminution de phosphorylation d'AKT (pAKT) chez des souris SMA (**Figures 6A et** 6B) par rapport aux souris contrôles saines (CTL VH) tandis que la phosphorylation d'ERK (pERK) augmente (**Figure 6C** **et** **6D**). Le traitement quotidien des souris SMA, pendant 11 jours avec BIO101, restaure partiellement cette balance dans la moelle épinière des animaux en augmentant le niveau de pAKT et en diminuant celui de pERK (**Figures 6B** **et** **6D**). De manière attendue dans ce modèle, la protéine SMN n'est pas exprimée chez les souris SMA. Comme observé dans le muscle *Plantaris,* le traitement avec BIO101 ne permet pas la restauration du niveau de SMN chez des souris SMA traitées pendant 11 jours (**Figures 6E et 6F**).

### d. Analyse des motoneurones

Une étude quantitative et qualitative des populations de motoneurones sur des coupes épaisses de la région lombaire de la moelle épinière (L1-L5) de souris contrôles saines ou SMA traitées avec le véhicule ou avec BIO101 pendant 11 jours a été réalisée par marquage immunofluorescent de la Choline AcétylTransférase (ChAT) tel que cela a été décrit précédemment (Biondi et al., 2008 ; Boyer et al., 2013). L'analyse a ensuite été raffinée par une étude des différentes sous-populations motoneuronales en caractérisant leur localisation dans la moelle épinière (position latérale ou médiale), ainsi que la distribution de la taille de leur soma.

Les souris anesthésiées ont été perfusées en intracardiaque avec du PBS. Les moelles épinières des souris ont été prélevées, fixées puis rincées. La région lombaire des moelle épinière (L1-L5) a été enrobée dans une solution d'agarose à 4%. Des sections transversales de 50 µm ont été réalisées à l'aide d'un vibratome sur toute la longueur de l'échantillon. Une section toutes les 5 sections de moelle épinière a été ensuite utilisée pour les analyses immunohistochimiques. Après une saturation en glycine 0,1M, les tissus ont ensuite été perméabilisés, bloqués puis marqués avec un anticorps primaire polyclonal de chèvre anti-choline acétyltransférase (ChAT) (1/400^{e}). Les coupes sont ensuite lavées puis incubées avec l'anticorps secondaire polyclonal anti-chèvre couplé à la Cyanine 3 (1:400^{e}). Les noyaux sont marqués à l'aide de Bisbenzimide (1/1000^{e}) puis les coupes sont lavées à nouveau avant d'être montées avec un inhibiteur du photoblanchiment des colorants fluorescents. La spécificité du marquage est vérifiée grâce à un marquage contrôle effectué en l'absence de l'anticorps primaire.

Les images ont été obtenues en utilisant une caméra montée sur un microscope à un grossissement x200 et couplée à une unité centrale de traitement de type microordinateur comprenant notamment un logiciel adéquat pour l'acquisition d'images. Tous les comptages ont été réalisés en utilisant un logiciel de traitement d'images.

En immunofluorescence, les motoneurones marqués avec l'anticorps anti-ChAT sont bien identifiables en gris clair sur les figures au niveau de la corne ventrale de la moelle épinière (**Figures 7A, 7B** **et** **7C**). Le nombre de motoneurones par coupe par hémi-moelle épinière ventrale a été déterminé pour chacun des trois groupes de souris. De manière attendue, le nombre de motoneurones (n=5) des souris SMA diminue significativement (**Figure 7B**) par rapport au nombre de motoneurones (n=5) retrouvé au niveau du groupe de souris saines contrôle (**Figure 7A**). En effet, cette dégénérescence motoneuronale est de 25% entre ces deux groupes (p<0,05)

**(****Figure 7D****).** De manière tout à fait intéressante, on observe qu'après 11 jours de traitement quotidien avec BIO101, le nombre de motoneurones est significativement plus important (p<0,05) chez ces souris SMA (**Figure 7C**) que chez les souris SMA traitées avec le véhicule (n=5). Ainsi, on observe que le traitement par BIO101 limite significativement la dégénérescence des motoneurones due à la pathologie et que le traitement exerce un effet neuroprotecteur significatif avec une perte des motoneurones qui se limite à 13% dans le groupe traité avec la molécule BIO101 (**Figure 7D**).

L'analyse quantitative a ensuite été raffinée, d'une part, en étudiant les différentes sous-populations motoneuronales en analysant leur localisation dans la moelle épinière (position latérale ou médiale), et d'autre part, en étudiant la distribution de la surface de leur soma.

On observe que le nombre de motoneurones latéraux (qui innervent les muscles distaux) est plus important chez des souris SMA traitées avec BIO101 par rapport à des souris SMA qui ont reçu le véhicule, tandis qu'il n'y a pas d'effet significatif sur les motoneurones médians (**Figure 7E**).

L'analyse de la surface du soma des motoneurones montre, tel qu'attendu, une atrophie des motoneurones chez les souris SMA traitées au véhicule par rapport aux souris contrôles saines avec une augmentation significative du nombre de motoneurones ayant une surface de leur soma inférieure à 600 µm² (p<0,05). Parallèlement à cela, on observe chez les souris SMA une perte des motoneurones ayant un soma d'une surface supérieure à 900 µm² (p<0,05) (**Figure 7F**)**.** Le traitement par BIO101 permet, quant à lui, de limiter cette atrophie des motoneurones en limitant de manière significative le nombre de petits motoneurones avec une surface de soma inférieure à 300 µm² (p<0,05) (**Figure 7F**)**.** Il est important de noter que, dans le cadre de la SLA, il a été décrit que les unités motrices ne sont pas affectées de manière homogène par le processus pathologique. En effet, le modèle murin pré-symptomatique de la maladie a mis en évidence la dégénérescence préférentielle des unités motrices de type FF (*Fast Fatiguable*) mettant en jeu des motoneurones avec un soma de grande surface (Pun et al., 2006). Une dégénérescence différentielle similaire a également été rapportée chez les patients (Dengler et al., 1990; Theys, Peeters et Robberecht, 1999). Ainsi, diminuer la proportion de motoneurones de petite surface au profit de ceux avec une surface de soma plus grande pourrait être une approche intéressante dans le cadre d'une pathologie telle que la SLA.

De manière complémentaire à l'analyse quantitative du nombre de motoneurones et de la taille de leur soma ainsi que de l'étude qualitative concernant leur localisation (médiane ou latérale dans la moelle épinière ventrale), nous avons étudié l'impact du traitement par BIO101 sur la protection des sous-populations de motoneurones (motoneurones lent, motoneurones intermédiaires et motoneurones rapide). Cette analyse qualitative des sous-populations de motoneurones a été réalisée sur des coupes épaisses de la région lombaire de la moelle épinière (L1-L5) de souris contrôles ou SMA traitées ou non avec BIO101 par marquage immunofluorescent de la Choline Acétyltransférase tel que cela a été décrit précédemment (Biondi et al., 2008 ; Branchu et al., 2013) ainsi qu'une analyse du type de motoneurones par des co-marquages en immunofluorescence avec l*'Estrogen-related recepteur-β* (ERRβ), marqueur spécifique des motoneurones lents, ou avec la *Matrix metallopeptidase 9* (MMP9), marqueur spécifique des motoneurones rapides.

Les souris anesthésiées ont été perfusées en intracardiaque avec du PBS. Les moelles épinières des souris ont été prélevées, fixées puis rincées. La région lombaire des moelles épinières (L1-L5) a été enrobée dans une solution d'agarose à 4%. Des sections transversales de 50 µm ont été réalisées à l'aide d'un vibratome sur toute la longueur de l'échantillon. Une section toutes les 5 sections de moelle épinière a été ensuite utilisée pour les analyses immunohistochimiques. Après une saturation en glycine 0,1M, les tissus ont ensuite été perméabilisés, bloqués puis marqués avec les anticorps primaires suivants : anticorps de chèvre anti-ChAT (1/400e), anticorps de souris anti-ERRβ (1/400^{e}), anticorps lapin anti-MMP9 (1/600^{e}). Après trois rinçages, les anticorps secondaires suivants sont incubés avec les coupes : anticorps d'âne anti-chèvre Cy5 (1/400^{e}), anticorps d'âne anti-souris Alexa 488 (1/400^{e}), anticorps d'âne anti-lapin Cy3 (1/400^{e}). Les noyaux sont marqués à l'aide de Bisbenzimide (1/1000e) puis les coupes sont lavées à nouveau avant d'être montées avec un inhibiteur du photoblanchiment des colorants fluorescents. La spécificité du marquage est vérifiée grâce à un marquage contrôle effectué en l'absence de l'anticorps primaire.

Les images ont été obtenues en utilisant une caméra montée sur un microscope à un grossissement x200 et couplée à une unité centrale de traitement de type microordinateur comprenant notamment un logiciel adéquat pour l'acquisition d'images. Tous les comptages ont été réalisés en utilisant un logiciel de traitement d'images.

Le nombre de motoneurones lents (ChAT+ ERRβ+) ne varie pas significativement dans les groupes, qu'il s'agisse des souris saines traitées avec le véhicule, des souris SMA traitées au véhicule ou avec BIO101 (**Figure 8**). Dans le groupe des animaux SMA traités avec le véhicule, on observe une perte significative du nombre moyen de motoneurones rapides (ChAT+ MMP9+) par rapport au groupe des souris contrôle saines (11 versus 16 respectivement; p<0,05). Cette perte se fait au bénéfice des motoneurones de type intermédiaire (ChAT+ ERRb- MMP9-) dont le nombre est significativement plus élevé dans le groupe de souris SMA véhicule en comparaison au nombre présent chez des souris saines contrôle (7 versus 4 ; p<0,05).

De manière intéressante, le traitement par BIO101 de souris SMA favorise préférentiellement la survie des motoneurones de type rapide (ChAT+ MMP9+). En effet, dans le groupe de souris SMA traitées avec BIO101, le nombre de motoneurones rapides est significativement plus élevé que dans le groupe de souris SMA ayant reçu le véhicule (14 versus 11 ; p<0,05).

Par conséquent, le traitement par BIO101 limite la perte motoneuronales observée dans ce modèle de SMA sévère, en particulier en protégeant les souris de la perte des motoneurones rapides.

### e. Analyse des jonctions neuromusculaires

La SMA est caractérisée par une altération spécifique des jonctions neuromusculaires induites par le manque de protéine SMN et par la dénervation induite par la mort spécifique des motoneurones (Kariya et al., 2008 ; Biondi et al., 2008 ; Chali et al., 2016). Nous avons réalisé une étude morphologique des jonctions neuromusculaires pour déterminer le degré de maturation et la fragmentation de la structure mature dite « en bretzel ». Pour cela, nous avons réalisé des marquages spécifiques des faces présynaptique (Synaptophysine et Neurofilament) et post-synaptique (α-bungarotoxine) par immunofluorescence (Leroy et al., 2014), sur des fibres musculaires dilacérées des muscles *soleus, plantaris* et *tibialis* des souris SMA traitées ou pas, avec la molécule BIO101.

Des coupes longitudinales de 75 µm d'épaisseur ont été réalisées au vibratome. Les coupes sont ensuite saturées en glycine 0,1M avec une faible agitation puis lavées au PBS. Elles sont ensuite bloquées et perméabilisées avec une solution de PBS-BSA 4%-serum de chèvre 5%-triton 0,5%. Les anticorps primaires anti-neurofilament (1/800^{e}) et anti-synaptophysine (SNAP25; 1/200^{e}) (pour identifier la face pré-synaptique de la jonction neuromusculaire) sont incubés pendant 48 heures et révélées avec un anticorps secondaire (Anti-lapin AlexaFluor^{®} 647; 1/400^{e}) puis lavées. Enfin, les coupes sont ensuite incubées avec un anti α-bungarotoxine directement couplé AlexaFluor^{®} 555 (1/500^{e}). Les coupes sont lavées, et les noyaux sont marqués au bis-benzimide (1/1000e) puis montées entre lame et lamelles avec un inhibiteur du photoblanchiment des colorants fluorescents pour une observation en imagerie microscopique en épifluorescence (**Figure 9A**). Les jonctions neuromusculaires sont définies et quantifiées selon trois catégories, de la plus immatures à la plus mature : plaque uniforme, perforée ou de forme « bretzel ».

Dans tous les muscles étudiés (Plantaris, Soleus, et Tibialis), le pourcentage de jonctions neuromusculaires uniformes chez des animaux SMA est très largement augmenté par rapport à des souris saines contrôle. Ce retard de maturation des jonctions neuromusculaires est attendu et a déjà été décrit dans la littérature (Biondi et al., 2008). En effet, dans le muscle extenseur rapide Plantaris, le pourcentage de jonctions neuromusculaires immatures à P10 est de 89,7% versus 49,7% chez des souris contrôles saines (p<0,05) (**Figure 9B**), dans le muscle extenseur lent Soleus, le pourcentage de jonctions neuromusculaires immatures est de 84,3% versus 45,7% chez des souris contrôles saines (p<0,05) (**Figure 9C**), dans le muscle fléchisseur rapide Tibialis, le pourcentage de jonctions neuromusculaires immatures est de 69,3% versus 28,7% chez des souris contrôles saines (p<0,05) (**Figure 9D**). Lorsque les souris SMA sont traitées quotidiennement avec BIO101, de leur naissance à P10, on observe une maturation plus importante des jonctions neuromusculaires dans tous les muscles testés, avec une diminution du pourcentage de plaques immatures au profit des plaque perforées, qui témoignent d'une accélération de la maturation. En effet, dans le muscle plantaris, 16,3% des jonctions sont de type perforé contre 10,4% chez des animaux SMA ayant reçu le véhicule (p=ns) (**Figure 9B**). Cette différence est significative dans les muscles Soleus (26,5% de jonctions perforées dans le groupe SMA traités contre 15,7% dans le groupe de souris SMA traitées avec le véhicule, p<0,05, **Figure 9C**) et dans le Tibialis (45,7% de jonctions perforées dans le groupe SMA traités contre 30,7% dans le groupe de souris SMA traitées avec le véhicule, p<0,05, **Figure 9D**). Ainsi, ces résultats montrent que le traitement par BIO101 accélère la maturation des jonctions neuromusculaires.

### f. Analyse des capacités motrices dans un modèle de souris SMA sévère

Des analyses phénotypiques des souris SMA sévère de type 2 traitées ou non à partir de P0 avec BIO101 ont été effectuées. Il a été effectué, de manière longitudinale tous les deux jours, un test des capacités motrices des souris de P5 à P9. Les inventeurs ont évalué les capacités de déplacement spontané par le test *open-field,* ainsi que la fatigabilité musculaire par *grip test,* tels que décrits précédemment (Biondi et al., 2008 ; Branchu et al., 2013 ; Chali et al., 2016).

Le dispositif utilisé pour test en *open-field* est différent en fonction de l'âge des souris. Pour des animaux de P0 à P6, il est constitué d'une boîte en plastique de 15 × 15 × 5 cm avec un quadrillage du champ divisé en 25 carreaux de 3 cm × 3 cm. Pour des animaux de P7 à P21, il est constitué d'une boîte en plastique de 28 × 28 × 5 cm avec un quadrillage du champ divisé en 16 carreaux de 7 cm × 7 cm. Les souris ont été testées individuellement et le dispositif d'évaluation a été lavé après chaque session. Chaque souris initialement placée au centre du champ a pu se déplacer librement pendant 5 minutes. Les mesures comportementales sont enregistrées par l'expérimentateur pendant ces 5 minutes et le nombre total de carreaux croisés a été relevé.

De manière attendue, à tous les temps testés (P5, P7 et P9) les souris SMA traitées avec le véhicule montrent des performances motrices significativement diminuées par rapport aux souris saines contrôles (**Figure 10A**). En effet, le nombre de carreaux qu'elles sont capables de croiser est de 10 à P5, 6 à P7 et 8 à P9 versus 17 à P5, 12 à P7 et 24 à P9 dans le groupe des souris saines contrôles (avec p<0,01, p<0,0001 et p<0,001 respectivement). A P5, le traitement par BIO101 des souris SMA n'améliore pas leurs performances motrices (11 carreaux) par rapport à des souris SMA traitées avec le véhicule (10 carreaux). A P7, la mobilité des souris traitées avec BIO101 augmente significativement (10 carreaux croisés) par rapport à des souris SMA ayant reçu le véhicule (6 carreaux croisés avec p<0,05). A P9, cette différence n'est pas significative mais le traitement avec BIO101 tend à avoir un effet bénéfique avec des souris qui sont capables de croiser 11 carreaux dans le groupe de souris SMA BIO101 par rapport à 8 carreaux dans le groupe SMA Véhicule, p=ns).

Pour évaluer la fatigabilité musculaire, la force de préhension des pattes antérieures des souris a été testée de P5 à P9 (*grip test*)*.* Les souris sont suspendues par leurs pattes antérieures à une fine tige métallique suspendue en l'air, de manière horizontale. Le temps passé à s'accrocher est relevé. Chaque souris a été soumise à cinq tentatives successives avec une période de repos d'une minute entre deux tests. Seul le meilleur essai est conservé pour l'évaluation des fonctions musculaires.

La fatigabilité musculaire, testée par le *grip test,* montre, de manière attendue, que les souris SMA traitées avec le véhicule ont une fonction musculaire significativement diminuée par rapport aux souris saines contrôle (**Figure 10B**). En effet, le temps durant lequel elles sont capables de rester suspendues à la tige métallique est de 0,2 secondes à P5, 6,2 secondes à P7 et 5,6 secondes à P9 versus 3,3 secondes à P5, 11,4 secondes à P7 et 16,1 secondes à P9 dans le groupe des souris saines contrôles (avec p<0,01, p<0,01 et p<0,001 respectivement). A P5, le traitement par BIO101 des souris SMA améliore significativement leurs performances musculaires (2,3 secondes) par rapport à des souris SMA traitées avec le véhicule (0,2 secondes ; p<0,05). A P7, le traitement par BIO101 tend à augmenter, mais de manière non significative, le temps d'accrochage des souris SMA par rapport à des souris SMA ayant reçu le véhicule (12,2 secondes versus 6,2 secondes respectivement avec p=ns). Enfin, à P9, BIO101 améliore très significativement ce paramètre chez les souris traitées avec BIO101 (20,9 secondes) par rapport à des souris SMA traitées avec le véhicule (5,6 secondes, avec p<0,01).

En conclusion, BIO101 possède des effets bénéfiques sur la motricité volontaire ainsi que sur la fatigabilité musculaire des animaux SMA.

### g. Analyse phénotypique des effets de BIO103

Le même modèle murin de type Il a été utilisé afin de caractériser les effets phénotypiques de la molécule BIO103 dans un modèle sévère de SMA. Les souris ont été traitées par gavage quotidien à une dose de 50mg/kg, soit avec la molécule BIO103 complexée au véhicule (dans le cas présent la cyclodextrine), soit avec le véhicule seul (VH). Le poids et la survie ont été analysés quotidiennement jusqu'à P11.

Les résultats démontrent que BIO103 (n=12), en traitement oral quotidien dès la naissance des souris, tend à limiter la perte de poids des animaux bien que la différence n'atteigne pas le seuil de significativité, en comparaison à des animaux traités avec le véhicule (n=22) (**Figure 11A**). En revanche, de manière significative (p<0,05), BIO103 augmente la survie pré-P11 des animaux traités (66,6% de survie à P11) par rapport à des souris ayant reçu le véhicule (36,3% de survie à P11) (**Figure 11B**)**.**

### f. Analyse des effets trophiques musculaires de BIO103

L'analyse histologique des coupes transversales colorées à l'Hématoxyline Eosine des muscles prélevés, montre un effet bénéfique de BIO103 sur l'atrophie musculaire (surface de section transversale des fibres musculaires).

De la même façon qu'avec la molécule BIO101, l'atrophie musculaire présente chez les souris SMA est limitée par le traitement, quelle que soient la nature et la typologie des muscles. En effet, le traitement par BIO103 permet de limiter significativement l'atrophie des trois muscles étudiés.

La quantification de la surface des fibres musculaires montre, de manière attendue, que cette atrophie est très marquée et est de 56,1% dans le *Tibialis* de souris SMA (n=4) par rapport à des souris contrôles (n=4). Cette atrophie est réduite significativement (p<0,05) à 22,1% à P11 grâce à un traitement avec BIO103 (n=4)

(**Figure 12A**)**.** Il en est de même pour le *Plantaris* pour lequel on observe une atrophie significative des fibres musculaires de 46% chez les souris SMA par rapport aux souris saines contrôles (p<0,05) et le traitement par BIO103 limite significativement cette atrophie à 15,3% (p<0,05) (**Figure 12B**) et dans le *Soleus* (50,2% pour des souris SMA comparées au contrôle (p<0,01) versus 37,1% avec un traitement avec BIO103, p<0,05) (**Figure 12C**)**.**

Afin de réaliser une analyse plus fine des effets de BIO103 sur l'atrophie, la distribution des fibres musculaires par catégories de surface de section transversale a été évaluée. La diminution significative de l'atrophie par la molécule BIO103 observée avec un traitement des souris SMA de P0 à P11, se traduit par un effet significatif de ce traitement sur la distribution des fibres selon leur surface de section transversale dans les trois muscles étudiés des souris SMA traitées avec BIO103 en comparaison avec les souris SMA traitées avec le véhicule (**Figures 13A****,** **13B et 13C**)**.** Dans le muscle *Tibialis* des souris SMA traitées avec BIO103 de P0 à P11, on observe une augmentation significative de la proportion de fibres ayant une surface de section transversale supérieure à 400µm² tandis que la proportion de petites fibres ayant une surface de section transversale inférieure à 200 µm² diminue significativement (p<0,05) par rapport au groupe de souris SMA véhicule (**Figure 13A**)**.** Dans le muscle *Plantaris* (**Figure 13B**) et dans le *Soleus* (**Figure 13C**) des SMA traitées avec BIO101 de P0 à P11, on observe une augmentation de la proportion de fibres musculaires ayant une surface de section transversale supérieure à 300 µm² accompagnée d'une diminution de celles ayant une surface de section transversale inférieure à 200 µm².

### 4. Conclusion

Compte tenu des propriétés de BIO101 et de BIO103 sur l'aplasie, l'atrophie musculaire et sur la dégénérescence des motoneurones de mammifères atteints par une amyotrophie spinale infantile, l'utilisation de phytoecdysones, et notamment de BIO101 et de BIO103 peut donc être proposée, seule ou en complément d'un traitement visant à corriger les effets d'une altération génique, pour préserver le tissu musculaire ainsi que les motoneurones, et ainsi ralentir l'évolution de maladies neuromusculaires qui ont pour conséquence la détérioration de fonction musculaire et/ou la perte motoneuronale. Les maladies neuromusculaires incluent notamment l'amyotrophie spinale.

De manière plus générale, il est à noter que les modes de mise en oeuvre et de réalisation de l'invention considérés ci-dessus ont été décrits à titre d'exemples non limitatifs et que d'autres variantes sont par conséquent envisageables.

### Références bibliographiques

Akten B, Kye MJ, Hao le T, Wertz MH, Singh S; et al. Interaction of survival of motor neuron (SMN) and HuD proteins with mRNA cpg15 rescues motor neuron axonal deficits. Proc. Natl. Acad. Sci. USA (2011); 108:10337-42.
Al-Chalabi A & Hardiman O. The epidemiology of ALS: a conspiracy of genes, environment and time. Nat. Rev. Neurol. (2013); 9:617-28.
Bevan AK, Hutchinson KR, Foust KD, Braun L, McGovern VL, et al. Early heart failure in the SMNDelta7 model of spinal muscular atrophy and correction by postnatal scAAV9-SMN delivery. Hum. Mol. Genet. (2010); 19:3895-905.
Biondi O, Grondard C, Lécolle S, Deforges S, Pariset C, et al. Exercise-induced activation of NMDA receptor promotes motor unit development and survival in a type 2 spinal muscular atrophy model mouse. J. Neurosci. (2008); 28:953-62.
Biondi O, Lopes P, Desseille C, Branchu J, Chali F, et al. Physical exercise reduces cardiac defects in type 2 spinal muscular atrophy-like mice. J. Physiol. (2012); 590:5907-25.
Boyer JG, Murray LM, Scott K, De Repentigny Y, Renaud JM, Kothary R. Early onset muscle weakness and disruption of muscle proteins in mouse models of spinal muscular atrophy. Skelet. Muscle (2013); 3:24.
Branchu J, Biondi O, Chali F, Collin T, Leroy F, et al. Shift from extracellular signal-regulated kinase to AKT/cAMP response element-binding protein pathway increases survival-motor-neuron expression in spinal-muscular-atrophy-like mice and patient cells. J. Neurosci. (2013); 33:4280-94.
Buhler D, Raker V, Luhrmann R & Fischer U. Essential rôle for the tudor domain of SMN in spliceosomal U snRNP assembly: implications for spinal muscular atrophy. Hum. Mol. Genet. (1999); 8:2351-7.
Chali, F. et al. Long-term exercise-specific neuroprotection in spinal muscular atrophy-like mice. J Physiol 594, 1931-1952 (2016).
Chiriboga CA, Swoboda KJ, Darras BT, lannaccone ST, Montes J, et al. Results from a phase 1 study of nusinersen (ISIS-SMN(Rx)) in children with spinal muscular atrophy. Neurology (2016); 86:890-7.
Cifuentes-Diaz C, Frugier T, Tiziano FD, Lacène E, Robiot N, et al. Deletion of murine SMN exon 7 directed to skeletal muscle leads to severe muscular dystrophy. J. Cell Biol. (2001); 152:1107-14.
Crawford TO & Pardo CA. The neurobiology of childhood spinal muscular atrophy. Neurobiol. Dis. (1996); 3:97-110.
Dengler R, Konstanzer A, Küther G, Hesse S, Wolf W, Struppler A. Amyotrophie lateral sclerosis: macro-EMG and twitch forces of single motor units. Muscle Nerve (1990); 13 (6):545-50.
Fidzianska A, Rafalowska J, Glinka Z. Ultrastructural study of motoneurons in Werdnig-Hoffmann disease. Clin. Neuropathol. (1984); 3:260-5.
Finkel RS, Chiriboga CA, Vaisar J, Day JW, Montes J, et al. Treatment of infantile-onset spinal muscular atrophy with nusinersen: a phase 2, open-label, dose-escalation study. Lancet (2016); 388:3017-26.
Frugier T, Tiziano FD, Cifuentes-Diaz C, Miniou P, Roblot N, et al. Nuclear targeting defect of SMN lacking the C-terminus in a mouse model of spinal muscular atrophy. Hum. Mol. Genet. (2000); 9:849-58.
Gorelick-Feldman J, MacLean D, Ilic N, Poulev A, Lila MA, Cheng D, Raskin I. Phytoecdysteroids increase protein synthesis in skeletal muscle cells. J. Agric. Food Chem. (2008); 56:3532-37.
Greensmith L & Vrbova G. Alterations of nerve-muscle interaction during postnatal development influence motoneurone survival in rats. Brain Res. Dev. Brain Res. (1992); 69(1):125-31 (1992).
Grice SJ & Liu JL. Survival motor neuron protein regulates stem cell division, prolifération, and différentiation in Drosophila. PLoS Genetics (2011) 7:e1002030. Hache M, Swoboda KJ, Sethna N, Farrow-Gillespie A, Khandji A, et al. Intrathecal Injections in Children With Spinal Muscular Atrophy: Nusinersen Clinical Trial Expérience. J. Child Neurol. (2016); 31:899-906.
Heier CR, Satta R, Lutz C, DiDonato CJ. Arrhythmia and cardiac defects are a feature of spinal muscular atrophy model mice. Hum. Mol. Genet. (2010); 19:3906-18.
Hsieh-Li HM, Chang JG, Jong YJ, Wu MH, Wang NM, et al. A mouse model for spinal muscular atrophy. Nat. Genet. (2000); 24:66-70.
Hua Y, Sahashi K, Hung G, Rigo F, Passini MA, et al. Antisense correction of SMN2 splicing in the CNS rescues necrosis in a type III SMA mouse model. Genes Dev. (2010); 24:1634-44.
Hunter G, Aghamaleky Sarvestany A, Roche SL, Symes RC, Gillingwater TH. SMN-dependent intrinsic defects in Schwann cells in mouse models of spinal muscular atrophy. Hum. Mol. Genet. (2014); 23:2235-50.
Hwee DT, Kennedy AR, Hartman JJ, Ryans J, Durham N, et al. The small-molecule fast skeletal troponin activator, CK-2127107, improves exercise tolérance in a rat model of heart failure. J. Pharmacol. Exp. Ther. (2015); 353:159-68.
Kariya S, Park GH, Maeno-Hikichi Y, Leykekhman O, Lutz C, Arkovitz MS et al. Reduced SMN protein impairs maturation of the neuromuscular junctions in mouse models of spinal muscular atrophy. Hum. Mol. Genet. (2008); 17(16):2552-69.
Kong L, Wang X, Choe DW, Polley M, Burnett BG, et al. Impaired synaptic vesicle release and immaturity of neuromuscular junctions in spinal muscular atrophy mice. J. Neurosci. (2009); 29:842-51.
Lawrence MM. Ajuga turkestanica as a countermeasure against sarcopenia and dynapenia. MS thesis, Appalachian State University (2012).
Lee Yl, Mikesh M, Smith I, Rimer M, Thompson W. Muscles in a mouse model of spinal muscular atrophy show profound defects in neuromuscular development even in the absence of failure in neuromuscular transmission or loss of motor neurons. Dev. Biol. (2011); 356:432-44.
Lefebvre S, Burlet P, Liu Q, Beltrandy S, Clermont O, et al. Correlation between severity and SMN protein level in spinal muscular atrophy. Nat. Genet. (1997); 16:265-9.
Leroy, F. et al. Early intrinsic hyperexcitability does not contribute to motoneuron degeneration in amyotrophie lateral sclerosis eLife. 3, e04046 (2014).
Liu Q, Fischer U, Wang F, Dreyfuss G. The spinal muscular atrophy disease gene product, SMN, and its associated protein SIP1 are in a complex with spliceosomal snRNP proteins. Cell (1997); 90:1013-21.
Lorson CL, Strasswimmer J, Yao JM, Baleja JD, Hahnen E, et al. SMN oligomerization defect correlates with spinal muscular atrophy severity. Nat. Genet. (1998); 19:63-6.
McWhorter ML, Monani UR, Burghes AH, Beattie CE. Knockdown of the survival motor neuron (Smn) protein in zebrafish causes defects in motor axon outgrowth and pathfinding. J. Cell Biol. (2003); 162:919-31.
Murray LM, Comley LH, Thomson D, Parkinson N, Talbot K, Gillingwater TH.. Selective vulnerability of motor neurons and dissociation of pre- and post-synaptic pathology at the neuromuscular junction in mouse models of spinal muscular atrophy. Hum. Mol. Genet. (2008); 17:949-62.
Nicole S, Desforges B, Millet G, Lesbordes J, Cifuentes-Diaz C, et al. Intact satellite cells lead to remarkable protection against Smn gene defect in differentiated skeletal muscle. J. Cell Biol. '2003); 161:571-82.
Passini MA, Bu J, Richards AM, Kinnecom C, Sardi SP, et al. Antisense oligonucleotides delivered to the mouse CNS ameliorate symptoms of sévère spinal muscular atrophy. Sci. Transl. Med. (2011); 3:72ra18.
Peter CJ, Evans M, Thayanithy V, Taniguchi-lshigaki N, Bach I, et al. The COPI vesicle complex binds and moves with survival motor neuron within axons. Hum. Mol. Genet. (2011); 20:1701-11.
Pun S, Santos AF, Saxena S, Xu L, Caroni P. Selective Vulnerability and Pruning of Phasic Motoneuron Axons in Motoneuron Disease Alleviated by CNTF. Nature Neuroscience (2006); 9 (3):408-19.
Rajendra TK, Gonsalvez GB, Walker MP, Shpargel KB, Salz HK, Matera AG. A Drosophila melanogaster model of spinal muscular atrophy reveals a function for SMN in striated muscle. J. Cell Biol. (2007); 176:831-41.
Rindt H, Feng Z, Mazzasette C, Glascock JJ, Valdivia D, et al. Astrocytes influence the severity of spinal muscular atrophy. Hum. Mol. Genet. (2015); 24:4094-102. Sanchez G, Dury AY, Murray LM, Biondi O, Tadesse H, et al. A novel function for the survival motoneuron protein as a translational regulator. Hum. Mol. Genet. (2013); 22:668-84.
Shababi M, Habibi J, Yang HT, Vale SM, Sewell WA, Lorson CL. Cardiac defects contribute to the pathology of spinal muscular atrophy models. Hum. Mol. Genet. (2010); 19:4059-71.
Shafey D, Cote PD, Kothary R. Hypomorphic Smn knockdown C2C12 myoblasts reveal intrinsic defects in myoblast fusion and myotube morphology. Exp. Cell Res. (2005); 311:49-61.
Simakin SYu, Panyushkin VV, Portugalov SN, Kostina LV, Martisorov EG. Combined application of préparation Ecdysten. Science Bulletin (1998); N°2, 29-31.
Somers E, Lees RD, Hoban K, Steigh JN, Zhou H, et al. Vascular Defects and Spinal Cord Hypoxia in Spinal Muscular Atrophy. Ann. Neurol. (2016); 79:217-30.
Syrov VN. Comparative experimental investigations of the anabolic activity of ecdysteroids and steranabols. Pharm. Chem. J. (2000); 34(4):193-197.
Theys PA, Peeters E, Robberecht W. Evolution of Motor and Sensory Déficits in Amyotrophic Lateral Sclerosis Estimated by Neurophysiological Techniques. J. Neurol. (1999); 246 (6):438-42.
Torres-Benito L, Neher MF, Cano R, Ruiz R, Tabares L. SMN requirement for synaptic vesicle, active zone and microtubule postnatal organization in motor nerve terminals. PLoS One (2011); 6:e26164.
Tóth N, Szabó A, Kacsala P, Héger J, Zádor E. 20-Hydroxyecdysone increases fiber size in a muscle-specific fashion in rat. Phytomedicine (2008); 15: 691-8.
Vitte J, Fassier C, Tiziano FD, Dalard C, Soave S, et al. Refined characterization of the expression and stability of the SMN gene products. Am J Pathol (2007); 171:1269-80.
Vitte JM, Davoult B, Roblot N, Mayer M, Joshi V, et al. Deletion of murine Smn exon 7 directed to liver leads to sévère defect of liver development associated with iron overload. Am. J. Pathol. (2004); 165:1731-41.
Vrbova G, Fisher TJ. The Effect of Inhibiting the Calcium Activated Neutral Protease, on Motor Unit Size after Partial Denervation of the Rat Soleus Muscle. Eur. J. Neurosci. (1989); 1:616-25.
Walker MP, Rajendra TK, Saieva L, Fuentes JL, Pellizzoni L, Matera AG. SMN complex localizes to the sarcomeric Z-disc and is a proteolytic target of calpain. Hum. Mol. Genet. (2008); 17:3399-410.
Zhang Z, Lotti F, Dittmar K, Younis I, Wan L, et al. SMN deficiency causes tissue-specific perturbations in the répertoire of snRNAs and widespread defects in splicing. Cell (2008); 133:585-600.

## Revendications

1. Composition comprenant au moins de la 20-hydroxyecdysone et/ou au moins un dérivé hémi-synthétique de 20-hydroxyecdysone pour son utilisation dans le traitement d'une affection spécifique des motoneurones chez le mammifère atteint de l'amyotrophie spinale infantile (SMA) ledit au moins un dérivé hémi-synthétique de 20-hydroxyecdysone étant choisi parmi :
- un composé de formule générale (I) : 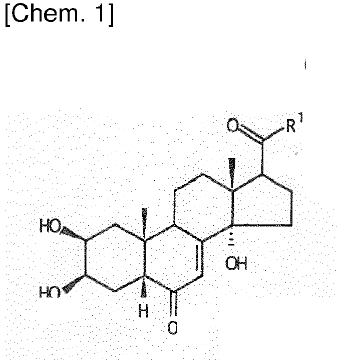 dans laquelle :
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆) ; un groupement CH₂Br ;
**W** étant un hétéroatome choisi parmi N, O et S, de préférence O et encore plus préférentiellement S.
- le composé de formule (II) :

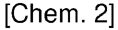

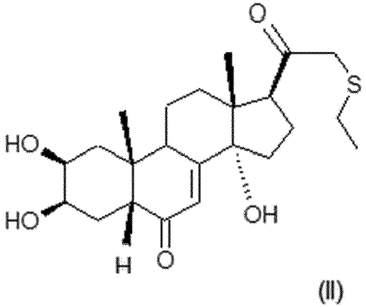

2. Composition pour son utilisation selon la revendication 1, dans laquelle la 20-hydroxyecdysone est sous forme d'extrait de plante ou d'une partie de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone.

3. Composition pour son utilisation selon la revendication 2, comportant de façon remarquable entre 0 et 0,05 %, en poids sec de l'extrait, d'impuretés susceptibles d'affecter l'innocuité, la disponibilité ou l'efficacité d'une application pharmaceutique dudit extrait.

4. Composition pour son utilisation selon l'une quelconque des revendications 2 à 3, dans laquelle la plante est choisie parmi *Stemmacantha carthamoides, Cyanotis arachnoidea* et *Cyanotis vaga.*

5. Composition pour son utilisation selon l'une quelconque des revendications 1 à 4, dans laquelle l'altération de la fonction musculaire est engendrée par une altération de la fonction des motoneurones ou leur dégénérescence.

6. Composition pour son utilisation selon l'une quelconque des revendications 1 à 5, dans laquelle l'affection des motoneurones résulte d'une altération génétique chez le mammifère atteint de la maladie neuromusculaire.

7. Composition pour son utilisation selon l'une quelconque des revendications 1 à 6, dans laquelle la fonction musculaire altérée est celle du muscle strié squelettique.

8. Composition pour son utilisation selon l'une quelconque des revendications 1 à 7, dans laquelle l'altération de la fonction musculaire est liée à une aplasie et/ou une atrophie.

9. Composition pour son utilisation selon l'une quelconque des revendications 1 à 8, dans laquelle la 20-hydroxyecdysone et/ou ledit au moins un dérivé hémi-synthétique de 20-hydroxyecdysone est utilisé pour traiter au moins une altération génétique responsable de la SMA.

10. Composition pour son utilisation selon l'une quelconque des revendications 1 à 9, dans laquelle la maladie neuromusculaire résulte d'une mutation du gène SMN1.

11. Composition pour son utilisation selon l'une quelconque des revendications 1 à 10, dans laquelle le traitement de l'affection spécifique des motoneurones comporte l'amélioration de la survie motoneuronale et/ou l'accélération de la maturation des jonctions neuromusculaires.

12. Composition pour son utilisation selon l'une quelconque des revendications 1 à 11, dans laquelle la 20-hydroxyecdysone et/ou ledit au moins un dérivé hémi-synthétique de 20-hydroxyecdysone est administré à une dose comprise entre 3 et 15 milligrammes par kilogramme par jour chez l'humain.

13. Composition pour son utilisation selon l'une quelconque des revendications 1 à 12, dans laquelle la 20-hydroxyecdysone et/ou ledit au moins un dérivé hémi-synthétique de 20-hydroxyecdysone est administré à une dose de 200 à 1000 mg/jour, en une ou plusieurs prises, chez un humain adulte, et une dose de 5 à 350 mg/jour, en une ou plusieurs prises, chez l'humain enfant ou le nourrisson.

14. Composition pour son utilisation selon l'une quelconque des revendications 1 à 13, dans laquelle ledit au moins un composé de formule générale (I) est choisi parmi :
- **n°1** : (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one;
- **n°2** : (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
- **n°3** : (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
- **n°4** : (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
- **n°5 :** (2S,3R,5R,10R,13R,14S,17S)-17-[2-(3-diméthylaminopropyl(méthyl)amino)acétyl]-2,3,14-trihydroxy-10,13-diméthyl-2,3,4,5,9,11,12,15,16,17-décahydro-1H-cyclopenta[a]phénanthrèn-6-one;
- **n°6** : 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
- **n°7** : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
- **n°8** : (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H-cyclopenta[a]phénanthrèn-6-one.

## Patentansprüche

1. Zusammensetzung, die mindestens 20-Hydroxyecdyson und/oder mindestens ein halbsynthetisches Derivat von 20-Hydroxyecdyson umfasst, zur Verwendung bei der Behandlung einer spezifischen Motoneuronenstörung bei Säugetieren, die an infantiler spinaler Amyotrophie (SMA) leiden, wobei mindestens ein halbsynthetisches Derivat von 20-Hydroxyecdyson ausgewählt ist aus:
- einer Verbindung der allgemeinen Formel (I): 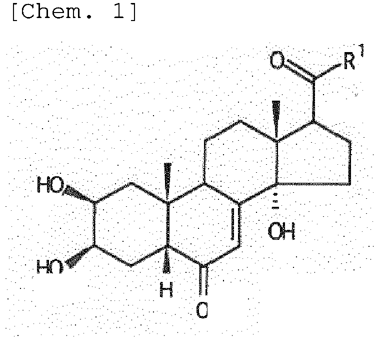 wobei:
R1 ausgewählt aus ist: einer (C₁-C₆)W(C₁-C₆)-Gruppe; einer (C₁-C₆)W(C₁-C₆)W(C₁-C₆)-Gruppe; einer (C₁-C₆)W(C₁-C₆)CO₂(C₁-C₆)-Gruppe; einer (C₁-C₆)A-Gruppe, wobei A einen Heterocyclus darstellt, der gegebenenfalls durch eine Gruppe vom Typ OH, OMe, (C₁-C₆), N(C₁-C₆), CO₂(C₁-C₆) substituiert ist; einer CH₂Br-Gruppe;
W ein Heteroatom ist, das aus N, O und S, vorzugsweise O und noch mehr bevorzugt S ausgewählt ist,
- die Verbindung der Formel (II):

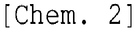

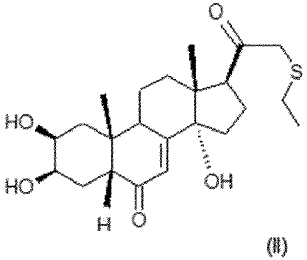

2. Zusammensetzung zur Verwendung nach Anspruch 1, wobei 20-Hydroxyecdyson in Form eines Pflanzenextrakts oder eines Pflanzenteils vorliegt, wobei die Pflanze aus Pflanzen ausgewählt ist, die mindestens 0,5 % 20-Hydroxyecdyson, bezogen auf das Trockengewicht der Pflanze, enthält, wobei der Extrakt mindestens 95 % und vorzugsweise mindestens 97 % 20-Hydroxyecdyson umfasst.

3. Zusammensetzung zur Verwendung nach Anspruch 2, wobei es erwähnenswert ist, dass die darin enthaltenen Verunreinigungen, die die Sicherheit, Verfügbarkeit oder Wirksamkeit der Arzneimittelanwendung des Extrakts beeinträchtigen können, 0 bis 0,05 %, bezogen auf das Trockengewicht des Extrakts, betragen.

4. Zusammensetzung zur Verwendung nach einem der Ansprüche 2 bis 3, wobei die Pflanze ausgewählt ist aus *Stemmacantha carthamoides, Cyanotis arachnoidea* und *Cyanotis vaga.*

5. Zusammensetzung zur Verwendung nach einem der Ansprüche 1 bis 4, wobei die Veränderung der Muskelfunktion durch eine Veränderung der Funktion von Motoneuronen oder deren Degeneration verursacht wird.

6. Zusammensetzung zur Verwendung nach einem der Ansprüche 1 bis 5, wobei sich die Störung der Motoneuronen aus einer genetischen Veränderung im Säugetier ergibt, das an der neuromuskulären Erkrankung leidet.

7. Zusammensetzung zur Verwendung nach einem der Ansprüche 1 bis 6, wobei die veränderte Muskelfunktion die der quergestreiften Skelettmuskulatur ist.

8. Zusammensetzung zur Verwendung nach einem der Ansprüche 1 bis 7, wobei die Veränderung der Muskelfunktion mit Aplasie und/oder Atrophie zusammenhängt.

9. Zusammensetzung zur Verwendung nach einem der Ansprüche 1 bis 8, wobei 20-Hydroxyecdyson und/oder das mindestens eine halbsynthetische Derivat von 20-Hydroxyecdyson zur Behandlung mindestens einer genetischen Veränderung verwendet wird, die für SMA verantwortlich ist.

10. Zusammensetzung zur Verwendung nach einem der Ansprüche 1 bis 9, wobei sich die neuromuskuläre Erkrankung aus einer Mutation des SMN1-Gens ergibt.

11. Zusammensetzung zur Verwendung nach einem der Ansprüche 1 bis 10, wobei die Behandlung der spezifischen Motoneuronenstörung die Verbesserung des Überlebens der Motoneuronen und/oder die Beschleunigung der Reifung der neuromuskulären Verbindungen umfasst.

12. Zusammensetzung zur Verwendung nach einem der Ansprüche 1 bis 11, wobei 20-Hydroxyecdyson und/oder das mindestens eine halbsynthetische Derivat von 20-Hydroxyecdyson dem Menschen in einer Dosis zwischen 3 und 15 Milligramm pro Kilogramm und Tag verabreicht wird.

13. Zusammensetzung zur Verwendung nach einem der Ansprüche 1 bis 12, wobei 20-Hydroxyecdyson und/oder das mindestens eine halbsynthetische Derivat von 20-Hydroxyecdyson einem erwachsenen Menschen in einer Dosis von 200 bis 1000 mg/Tag in einer oder mehreren Dosen und einem menschlichen Kind oder einem Säugling in einer Dosis von 5 bis 350 mg/Tag in einer oder mehreren Dosen verabreicht wird.

14. Zusammensetzung zur Verwendung nach einem der Ansprüche 1 bis 13, wobei die mindestens eine Verbindung der allgemeinen Formel (I) ausgewählt ist aus:
- Nr. 1: (2S,3R,5R,10R,13R,145,17S)-2,3,14-Trihydroxy-10,13-dimethyl-17-(2-Morpholinoacetyl)-2, 3, 4, 5, 9, 11, 12, 15, 16, 17-decahydro-1H-cyclopenta[a]phenanthren-6-on;
- Nr. 2: (2S,3R,5R,10R,13R,14S,17S)-2,3,14-Trihydroxy-17-[2-(3-Hydroxypyrrolidin-1-yl)acetyl]-10,13-dimethyl-2, 3, 4, 5, 9, 11, 12, 15, 16, 17-decahydro-1H-cyclopenta[a]phenanthren-6-on;
- Nr. 3: (2S,3R,5R,10R,13R,14S,17S)-2,3,14-Trihydroxy-17-[2-(4-hydroxy-1-piperidyl)acetyl]-10,13-dimethyl-2, 3, 4, 5, 9, 11, 12, 15, 16, 17-decahydro-1H-cyclopenta[a]phenanthren-6-on;
- Nr. 4: (2S,3R,5R,10R,13R,14S,17S)-2,3,14-Trihydroxy-17-[2-[4-(2-hydroxyethyl)-1-piperidyl]acetyl]-10,13-dimethyl-2, 3, 4, 5, 9, 11, 12, 15, 16, 17-decahydro-1H-cyclopenta[a]phenanthren-6-on;
- Nr. 5: (2S,3R,5R,10R,13R,14S,17S)-17-[2-(3-Dimethylaminopropyl(methyl)amino)acetyl]-2,3,14-trihydroxy-10, 13-dimethyl-2, 3, 4, 5, 9, 11, 12, 15, 16, 17-decahydro-1H-cyclopenta[a]phenanthren-6-on;
- Nr. 6: 2-[2-Oxo-2-[(2S,3R,5R,10R,13R,14S,17S)-2,3,14-trihydroxy-10, 13-dimethyl-6-oxo-2, 3, 4, 5, 9, 11, 12, 15, 16, 17-decahydro-1H-cyclopenta[a]phenanthren-17-yl]ethyl]sulfanylessigsäureethylester;
- Nr. 7: (2S,3R,5R,10R,13R,14S,17S)-17-(2-Ethylsulfanylacetyl)-2,3,14-trihydroxy-10,13-dimethyl-2, 3, 4, 5, 9, 11, 12, 15, 16, 17-decahydro-1H-cyclopenta[a]phenanthren-6-on;
- Nr. 8: (2S,3R,5R,10R,13R,14S,17S)-2,3,14-Trihydroxy-17-[2-(2-hydroxyethylsulfanyl)acetyl]-10,13-dimethyl-2, 3, 4, 5, 9, 11, 12, 15, 16, 17-decahydro-1H-cyclopenta[a]phenanthren-6-on.

## Claims

1. Composition comprising at least 20-hydroxyecdysone and/or at least one semi-synthetic derivative of 20-hydroxyecdysone for the use thereof in the treatment of a specific disorder of the motoneurons in mammals suffering from infantile spinal muscular atrophy (SMA), said at least one semi-synthetic derivative of 20-hydroxyecdysone being chosen from:
- a compound of general formula (I): 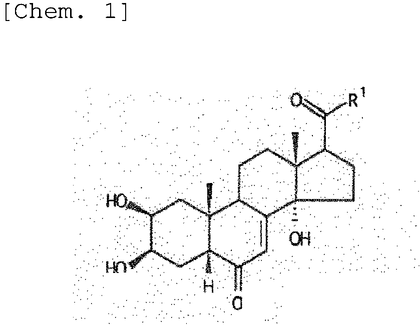 wherein:
**R¹** is chosen from: a (C₁-C₆)**W**(C₁-C₆) group; a (C₁-C₆)**W**(C₁-C₆)**W**(C₁-C₆) group; a (C₁-C₆)**W**(C₁-C₆)CO₂(C₁-C₆) group; a (C₁-C₆)**A** group, **A** representing a heterocycle optionally substituted with a group of the OH, OMe, (C₁-C₆), N(C₁-C₆), CO₂(C₁-C₆) type; a CH₂Br group;
**W** being a heteroatom chosen from N, O and S, preferably O and even more preferably S.
- the compound of formula (II):

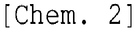

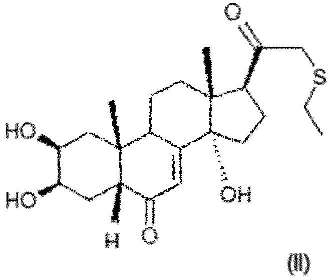

2. Composition for the use thereof according to claim 1, wherein 20-hydroxyecdysone is in the form of a plant extract or an extract of a plant part, said plant being chosen from plants containing at least 0.50 of 20-hydroxyecdysone by dry weight of said plant, said extract including at least 95%, and preferably at least 97%, of 20-hydroxyecdysone.

3. Composition for the use thereof according to claim 2, including remarkably between 0 and 0.05%, by dry weight of the extract, of impurities likely to affect the safety, the availability or the efficacy of a pharmaceutical application of said extract.

4. Composition for the use thereof according to any of claims 2 to 3, wherein the plant is chosen from *Stemmacantha carthamoides, Cyanotis arachnoidea* and *Cyanotis vaga.*

5. Composition for the use thereof according to any of claims 1 to 4, wherein the alteration of the muscular function is generated by an alteration of the motoneurons function or the degeneration thereof.

6. Composition for the use thereof according to any of claims 1 to 5, wherein the disorder of the motoneurons results from a genetic alteration in mammals suffering from neuromuscular disease.

7. Composition for the use thereof according to any of claims 1 to 6, wherein the altered muscular function is that of striated skeletal muscle.

8. Composition for the use thereof according to any of claims 1 to 7, wherein the alteration of the muscular function is linked to aplasia and/or atrophy.

9. Composition for the use thereof according to claim 1 to 8, wherein 20-hydroxyecdysone and/or said at least one semi-synthetic derivative of 20-hydroxyecdysone is used to treat at least one genetic alteration responsible for SMA.

10. Composition for the use thereof according to any of claims 1 to 9, wherein the neuromuscular disease results from a mutation of the SMN1 gene.

11. Composition for the use thereof according to any of claims 1 to 10, wherein the treatment of the specific disorder of the motoneurons includes the improvement in motoneuron survival and/or the acceleration in the maturation of the neuromuscular junctions.

12. Composition for the use thereof according to any of claims 1 to 11, wherein 20-hydroxyecdysone and/or said at least one semi-synthetic derivative of 20-hydroxyecdysone is administered at a dose comprised between 3 and 15 milligrams per kilogram per day in humans.

13. Composition for the use thereof according to any of claims 1 to 12, wherein 20-hydroxyecdysone and/or said at least one semi-synthetic derivative of 20-hydroxyecdysone is administered at a dose of 200 to 1000 mg/day, divided into one or more doses, in human adults, and a dose of 5 to 350 mg/day, divided into one or more doses, in human children or infants.

14. Composition for the use thereof according to any of claims 1 to 13, wherein said at least one compound of the general formula (I) is chosen from:
- **no. 1:** (2S,3R,5R,10R,13R,14S,17S)-2,3,14-trihydroxy-10,13-dimethyl-17-(2-morpholinoacetyl)-2, 3, 4, 5, 9, 11, 12, 15, 16, 17-decahydro-1H-cyclopenta[a]phenanthren-6-one;
- **no. 2:** (2S,3R,5R,10R,13R,14S,17S)-2,3,14-trihydroxy-17-[2-(3-hydroxypyrrolidin-1-yl)acetyl]-10,13-dimethyl-2, 3, 4, 5, 9, 11, 12, 15, 16, 17-decahydro-1H-cyclopenta[a]phenanthren-6-one;
- **no. 3:** (2S,3R,5R,10R,13R,14S,17S)-2,3,14-trihydroxy-17-[2-(4-hydroxy-1-piperidyl)acetyl]-10,13-dimethyl-2, 3, 4, 5, 9, 11, 12, 15, 16, 17-decahydro-1H-cyclopenta[a]phenanthren-6-one;
- **no. 4:** (2S,3R,5R,10R,13R,14S,17S)-2,3,14-trihydroxy-17-[2-[4-(2-hydroxyethyl)-1-piperidyl]acetyl]-10,13-dimethyl-2, 3, 4, 5, 9, 11, 12, 15, 16, 17-decahydro-1H-cyclopenta[a]phenanthren-6-one;
- **no. 5:** (2S,3R,5R,10R,13R,14S,17S)-17-[2-(3-dimethylaminopropyl(methyl)amino)acetyl]-2,3,14-trihydroxy-10, 13-dimethyl-2, 3, 4, 5, 9, 11, 12, 15, 16, 17-decahydro-1H-cyclopenta[a]phenanthren-6-one;
- **no. 6:** 2-[2-oxo-2-[(2S,3R,5R,10R,13R,14S,17S)-2,3,14-trihydroxy-10, 13-dimethyl-6-oxo-2, 3, 4, 5, 9, 11, 12, 15, 16, 17-decahydro-1H-cyclopenta[a]phenanthren-17-yl]ethyl]ethyl sulfanylacetate;
- **no. 7:** (2S,3R,5R,10R,13R,14S,17S)-17-(2-ethylsulfanylacetyl)-2,3,14-trihydroxy-10,13-dimethyl-2,3,4,5,9,11,12,15,16,17-decahydro-1H-cyclopenta[a]phenanthren-6-one;
- **no. 8:** (2S,3R,5R,10R,13R,14S,17S)-2,3,14-trihydroxy-17-[2-(2-hydroxyethylsulfanyl)acetyl]-10,13-dimethyl-2,3,4,5,9,11,12,15,16,17-decahydro-1H-cyclopenta[a]phenanthren-6-one.
